# Europäisches Patentamt
## European Patent Office
### Office européen des brevets

(19)

(11) Veröffentlichungsnummer : **0 067 425**
**B1**

(12) **EUROPÄISCHE PATENTSCHRIFT**

(45) Veröffentlichungstag der Patentschrift :
18.09.85

(21) Anmeldenummer : 82105097.8

(22) Anmeldetag : 11.06.82

(51) Int. Cl.⁴ : **C 07 K 5/08, C 07 K 5/10,**
**C 07 K 7/06, A 61 K 31/02**

(54) Gegebenenfalls geschützte Peptide, Verfahren zu ihrer Herstellung und diese enthaltende Arzneimittel.

(30) Priorität : 12.06.81 HU 175581

(43) Veröffentlichungstag der Anmeldung :
22.12.82 Patentblatt 82/51

(45) Bekanntmachung des Hinweises auf die Patenterteilung : 18.09.85 Patentblatt 85/38

(84) Benannte Vertragsstaaten :
BE CH DE FR GB IT LI NL SE

(56) Entgegenhaltungen :
EP-A- 0 018 182
EP-A- 0 025 897
GB-A- 1 565 032
CHEMICAL PHARMACEUTICAL BULLETIN, Band 28, Nr. 8, 1980, Seiten 2507-2511, The Chemical Society of Japan, Tokyo, JP. T. ABIKO et al.: "The effect of thymopoietin II fragments and their analogs on E-rosette forming cells in the uremic state"

(73) Patentinhaber : RICHTER GEDEON VEGYESZETI GYAR R.T.
Gyömröi ut 19-21
H-1475 Budapest X (HU)

(72) Erfinder : Kisfaludy, Lajos, Dr.
Riadò u. 6/a
Budapest II (HU)
Erfinder : Nyeki, geb. Kuprina, Olga
Ungvár u. 56/g
Budapest XIV (HU)
Erfinder : Schön, Istvan, Dr.
Boglár u. 19
Budapest X (HU)
Erfinder : Dénes, Lászlò, Dr.
Szél u. 19
Budapest III (HU)
Erfinder : Ember, Julia, Dr.
Fülemüle u. 12-18 5/b
Budapest XII (HU)
Erfinder : Hajòs, György, Dr.
Gábor A. u. 59
Budapest II (HU)
Erfinder : Szporny, Lászlò, Dr.
Szabolcska M. u. 7
Budapest XI (HU)
Erfinder : Szende, Béla, Dr.
Üllöi ut 55
Budapest IX (HU)

(74) Vertreter : Beszédes, Stephan G. Dr.
Münchener Strasse 80a Postfach 1168
D-8060 Dachau (DE)

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 0 067 425

**·Beschreibung**

Die Erfindung betrifft gegebenenfalls geschützte, Peptide, ein Verfahren zu ihrer Herstellung und diese enthaltende Arzneimittel, insbesondere solche zur Beeinflussung der Immunregulation.

Die in den letzten Jahren vorgenommenen Forschungen haben eindeutig bewiesen, daß die Thymushormone an den das von den T-Lymphozyten abhängende immunologische Gleichgewicht regulierenden Vorgängen unmittelbar teilnehmen. Wie anzunehmen ist, bewirken diese Hormone die Umbildung der jungen T-Zellen zu reifen differenzierten Zellen, die dann in der von Zellen vermittelten Immunität und in der Regulierung der unterschiedlichen Arten der Immunantworten eine zentrale Rolle spielen. Die Thymushormone wirken nicht nur im Thymus, sondern auch außerhalb dessen, sie haben Anteil an der Bildung der cytotoxischen, der Hilfs- und Unterdrückerzellen (Helper- und Supressorzellen). An immundifizienten und krebskranken Patienten mit Thymusextrakten vorgenommene klinische Untersuchungen gaben der Forschung weiteren Schwung. Mit der Entwicklung der Verfahren konnte ferner festgestellt werden, daß der Spiegel der Thymushormone im Serum zum Beispiel bei DiGeorge-Syndromen, IgA-Mangel und allgemeiner Hauttuberkulose absinkt, im Falle rheumatoider Arthritis hingegen ansteigt. Die Vermutung liegt daher nahe, daß durch Regulierung des Hormonspiegels diese Krankheiten in günstiger Weise beeinflußt werden können (E. Arrigeni-Martelli : Drugs of Today *16* [1980], 203).

Es ist bereits gelungen, mehrere Thymushormone als Reinsubstanzen zu isolieren, ihre Struktur aufzuklären und sie sogar zu synthetisieren. Zu diesen Hormonen gehört das aus 49 Aminosäuren aufgebaute Thymopoietin (Goldstein und Mitarbeiter : Ann. N.Y. Acad. Sci. *249* [1975], 177 ; US-Patentschriften 4 002 740 und 4 071 949). Mit natürlichen und später auch mit synthetischen Produkten (Fujino und Mitarbeiter : Chem. Pharm. Bull. *25* [1977], 1 486 ; Blisnakow und Mitarbeiter : Biochem. Biophys. Res. Commun. *80* [1978], 631) konnte eindeutig nachgewiesen werden, daß das Thymopoietin in vitro und in vivo die Differenzierung der T-Zellen induziert.

Da die von natürlichen Quellen herstammende Substanz zur Deckung des Bedarfes nicht ausreichend ist und der mit dem synthetischen Produkt verbundene hohe Aufwand die praktische Anwendung hindert, richtete sich die Forschung bald darauf, das aktive Zentrum des Thymopoietines zu finden. Zuerst wurde von einem Tridecapeptid, dem Thymopoietin-(24-41) (Schlesinger und Mitarbeiter : Cell. *5* [1978], 631), dann auch von einem als TP5 bezeichneten Pentapeptid, dem Thymopoietin-(32-36), das heißt L-Arginyl-L-lysyl-L-asparagyl-L-valyl-L-tyrosin, festgestellt, daß sie sämtliche Wirkungen des kompletten Hormones aufweisen (Goldstein und Mitarbeiter : Science *204* [1979], 1 309 ; US-Patentschrift 4 190 646). Neuerdings wurden auch einzelne Fragmente und Analoge des L-Arginyl-L-lysyl-L-asparagyl-L-valyl-L-tyrosines beschrieben, diese erwiesen sich jedoch im untersuchten System als nicht wirksam (Abiko und Mitarbeiter : Chem. Pharm. Bull. *28* [1980], 2 507).

Ferner sind in der britischen Patentschrift 1 565 032 das Polypeptid L-Arginyl-L-lysyl-L-asparagyl-L-valyl-L-tyrosin und Derivate desselben sowie ihre pharmakologische Wirkung zur Beeinflussung der Immunregulation beschrieben. Alle in dieser Druckschrift enthaltenen Verbindungen sind aus mindestens 5 Aminosäureeinheiten aufgebaut. Vom ersten Erfinder der genannten britischen Patentschrift 1 565 032, Gideon Goldstein, wurde in dessen Artikel (mit den Mitverfassem T. Audhya und G. A. Heavner) in Peptides, Proc. 7th Amer. Peptide Symp. Eds. D. H. Rich and E. Gross, Pierce Chem. Comp. 1981, Seiten 535 bis 539 auf Seite 536, Zeilen 1 bis 3 noch 3 Jahre nach dem Anmeldetag der britischen Patentschrift 1 565 032 die Feststellung gemacht, daß das Pentapeptid die kleinste Fraktion, welche T-Zellendifferenzierungsaktivität zeigt, ist.

In der britischen Patentschrift 1 565 032 ist als biologische Wirkung von deren Verbindungen das Induzieren der Differenzierung der T-Zelle in Konzentrationen von 1 ng bis 10 μg/ml angegeben. Dies bedeutet einen Bereich von 4 Größenordnungen.

Weiterhin sind aus der europäischen Patentanmeldung mit der Veröffentlichungsnummer 18 182 Pentapeptide der Formel A-X-Z-Y-B, bei welchen A unter anderem für einen Arginylrest stehen kann, X unter anderem einen Lysylrest oder einen Alanylrest bedeuten kann, Z unter anderem einen Asparagylrest oder einen Glutamylrest darstellen kann, Y unter anderem für einen Valylrest oder einen Isoleucylrest stehen kann und B einen Tyrosylrest oder einen von diesem abgeleiteten Rest darstellt, sowie ihre pharmakologische Wirkung zur Beeinflussung der Immunregulation bekannt. Dabei liegt aber als fünfte Aminosäureeinheit ein Rest von L-Tyrosin vor. Das Wirkungsspektrum der aus der europäischen Patentanmeldung mit der Veröffentlichungsnummer 18 182 bekannten Verbindungen ist mit dem des L-Arginyl-L-lysyl-L-asparagyl-L-valyl-L-tyrosines identisch. Dabei sind aber für einzelne Verbindungen keine biologischen Werte angegeben.

Außerdem sind aus der europäischen Patentanmeldung mit der Veröffentlichungsnummer 25 897 Pentapeptide der allgemeinen Formel A-B-S-X-Y, bei welchen A unter anderen für einen L-Arginyl-, L-Lysyl- oder Pyroglutamylrest stehen kann, B unter anderen einen L-Lysyl- oder L-Arginylrest bedeuten kann, S unter anderen einen L-Glutamylrest darstellen kann, X einen L-Valyl- oder L-Isoleucylrest bedeutet und Y einen Rest einer L- beziehungsweise D-Aminosäure mit hydrophober Seitenkette darstellen kann, bekannt. In dieser Druckschrift ist erwähnt, daß zur thymusähnlichen Wirkung der basisch-basisch-sauer-hydrophob-hydrophobe Charakter erforderlich ist. Ferner wird in dieser Druck-

2

schrift bemerjkt, daß, insbesondere im Falle, daß die saure Aminosäure D-Glutaminsäure oder D-α-Aminoadipinsäure ist, die Peptide in Leberhomogenaten gegenüber den natürlichen Thymuspeptiden eine wesentlich verlängerte Lebensdauer besitzen. Dazu wird auch bemerkt, daß diese Substitution auch deswegen gut sei, weil die Asparaginsäurepeptide nicht stabil sind, indem sie sich leicht über Asparaginimid-Peptide in Isoasparaginpeptide umlagern können. Zwar sind Prüfverfahrensweisen angegeben, es ist jedoch für keine einzige der genannten 40 Verbindungen irgendein biologischer Wert angegeben.

Der Erfindung liegt daher die Aufgabe zugrunde, weniger Aminosäureeinheiten aufweisende neue Peptide mit überlegenen pharmakologischen, insbesondere die Immunregulation beeinflussenden, Wirkungen, ein Verfahren zur Herstellung derselben und diese Verbindungen enthaltende Arzneimittel zu schaffen.

Es wurde nun überraschenderweise festgestellt, daß, wenn mit der Kettenverkürzung des L-Arginyl-L-lysyl-L-asparagyl-L-valyl-L-tyrosines nicht am N-endständigen Teil begonnen wird, wie dies von den japanischen Forschern getan wurde (Abiko und Mitarbeiter, siehe oben), sondern am C-endständigen Teil, sich ergab, daß nicht nur das Tetrapeptid L-Arginyl-L-lysyl-L-asparagyl-L-valin, sondern sogar das Tripeptid L-Arginyl-L-lysyl-L-asparaginsäure aktiv ist, die Anzahl der E-Rosetten bildenden Lymphozyten erhöht. Die vorliegende Erfindung betrifft nun die genannten Tetra- und Tripeptid und einige ihrer Analogen sowie die Synthese dieser Peptide. Unter den Analogen wurden überraschenderweise auch Derivate, welche eine entgegengesetzte biologische Wirkung zeigten, festgestellt. Dies ist der erste Beweis dafür, daß die die Immunantwort auslösende und die sie hemmende Wirkung auf einander strukturell sehr nahestehende Elemente zurückgehen.

Gegenstand der Erfindung sind daher

Peptide der Formeln

|  |  |
|---|---|
| Arg-Lys-Asp | I, |
| Arg-Lys-Asp-Val | II, |
| Arg-Lys-Asn-Val | III, |
| Arg-Lys-Asu-Val | IV, |
| Arg-Lys-Ala-Val | V, |
| Arg-Lys-Asp-Ala | VI, |
| Arg-Lys-Asp-Ile | VII, |
| Arg-Lys-Glu-Val | VIII, |
| Arg-Ala-Asp-Val | IX, |
| Arg-Asp-Lys-Val | X, |
| Ala-Lys-Asp-Val | XI, |
| Lys-Arg-Asp-Val | XII, |
| < Glu-Arg-Lys-Asp | XIII, |
| < Glu-Arg-Lys-Asp-Val | XIV, |
| < Glu-Arg-Lys-Asp-Val-Tyr | XV, |

sowie deren Salze, Komplexe, Amide und Alkylester mit 1 bis 5 Kohlenstoffatom(en) im Alkylteil derselben, gegebenenfalls substituiert mit in der Peptidchemie üblicherweise verwendeten Schutzgruppen.

Vorzugsweise sind die Alkylester der erfindungsgemäßen Peptide solche mit 1 bis 4, insbesondere 1 oder 2 Kohlenstoffatom(en) im Alkylteil.

Unter Komplexen der erfindungsgemäßen Peptide sind pharmazeutisch brauchbare Komplexe mit organischen oder anorganischen Verbindungen, durch welche eine verzögerte Wirkung beziehungsweise Retardwirkung der Peptide gewährleistet ist, zu verstehen. Als organische Verbindungen sind zum Beispiel Gelatine, Carboxymethylcellulosen, Alginsäureester, Poly-(phloretinphosphate), Aminosäurepolymere und -copolymere geeignet. Als anorganische Verbindungen kommen beispielsweise Hydroxyde beziehungsweise schwerlösliche Salze, wie Phosphate, von Metallen, insbesondere von Zink, in Frage.

Soweit in Peptiden der europäischen Patentanmeldung mit der Veröffentlichungsnummer 18 182 die ersten 4 Aminosäureeinheiten in derselben Folge gleich wie bei erfindungsgemäßen Peptiden vorliegen, unterscheiden sich diese von den ersteren darin, daß sie nicht den Rest von L-Tyrosin als fünfte Aminosäureeinheit aufweisen können.

Insofern als in Peptiden der europäischen Patentanmeldung mit der Veröffentlichungsnummer 25 897 die ersten 4 Aminosäureeinheiten in derselben Folge gleich wie bei erfindungsgemäßen Peptiden vorliegen, unterscheiden sich diese von den ersteren darin, daß sie nicht den Rest von L-Tyrosin beziehungsweise L-Tryptophan als fünfte Aminosäureeinheit aufweisen können. Gemäß den allgemeinen Festlegungen im Patentanspruch 1 der genannten Druckschrift kann die fünfte Aminosäureeinheit nicht nur ein Rest von L-Tyrosin beziehungsweise L-Tryptophan sein, sondern hierfür kommen auch eine Reihe von anderen Aminosäureresten in Frage. Wesentlich ist aber, daß stets eine fünfte Aminosäureeinheit vorliegt, durch deren Fehlen sich die erfindungsgemäßen Peptide der Formeln I bis XIII von den Peptiden der genannten Druckschrift auf jeden Fall unterscheiden, während die erfindungsgemäßen Peptide der Formeln XIV und XV in der genannten Druckschrift nicht beschrieben sind, weil im Gegensatz zu diesen in den Peptiden der genannten Druckschrift die dritte Aminosäureeinheit von einem L-Lysylrest verschieden ist und die vierte Aminosäureeinheit kein L-Asparagylrest sein kann.

Gegenüber der Tatsache, daß in der britischen Patentschrift 1 565 032 und in den europäischen Patentanmeldungen mit den Veröffentlichungsnummern 18 182 und 25 897 als kleinste Peptide Pentapeptide beschrieben sind, ist die erfindungsgemäße Feststellung, daß auch kleinere Fragmente als das L-Arginyl-L-lysyl-L-asparagyl-L-valyl-L-tyrosin [TP5], und zwar solche mit 3 oder 4 Aminosäureeinheiten [TP3 beziehungsweise TP4] auf die Thymushormone wertvolle biologische Wirkungen ausüben, überraschend. Zwar sind in Chemical Pharmaceutical Bulletin, Band 28, Nr. 8, 1980, Seiten 2 507 bis 2 511 auch kürzere Fragmente (Di-, Tri- und Tetrapeptide) genannt, diese zeigten aber keine biologische Wirksamkeit.

Gegenstand der Erfindung ist auch ein Verfahren zur Herstellung der erfindungsgemäßen Verbindungen, welches dadurch gekennzeichnet ist, daß in in der Peptidchemie an sich bekannter Weise aus den entsprechenden geschützten Aminosäuren beziehungsweise ihren Salzen, Komplexen, Amiden beziehungsweise Alkylestern die geschützten Peptide beziehungsweise ihre Salze, Komplexe, Amide beziehungsweise Alkylester synthetisiert und in an sich bekannter Weise gegebenenfalls von diesen die Schutzgruppe(n) abgespalten wird beziehungsweise werden und/oder gegebenenfalls die, gegebenenfalls geschützten, Peptide der allgemeinen Formeln I, II, III, IV, V, VI, VII, VIII, IX, X, XI, XII, XIII, XIV beziehungsweise XV in Salze oder Komplexe überführt beziehungsweise aus den Salzen die freien Basen freigesetzt und/oder sie in andere Salze überführt werden. Dabei sind die erfindungsgemäßen geschützten Peptide einschließlich ihrer Salze, Komplexe, Amide und Alkylester wertvolle Zwischenprodukte zur Herstellung der entsprechenden erfindungsgemäßen freier Peptide einschließlich ihrer Salze, Komplexe, Amide und Alkylester.

Zweckmäßig wird das erfindungsgemäße Verfahren in der Weise durchgeführt, daß die C-endständige Aminosäure beziehungsweise ein, gegebenenfalls an der Seitenfunktion geschütztes, die C-endständige Aminosäure aufweisendes Peptidfragment des herzustellenden Peptides beziehungsweise Salzes, Komplexes, Amides beziehungsweise Alkylesters desselben beziehungsweise ein Amid beziehungsweise ein Alkylester mit 1 bis 5 Kohlenstoffatom(en) beziehungsweise ein an der Carboxylgruppe in sonstiger Weise geschütztes Derivat dieser C-endständigen Aminosäure beziehungsweise dieses Peptidfragmentes mit einem an ihrem beziehungsweise seinem endständigen Stickstoffatom und gegebenenfalls an der Seitenfunktion geschützten und an der Carboxylgruppe aktivierten Derivat der in der Aminosäuresequenz des herzustellenden Peptides beziehungsweise Salzes, Komplexes, Amides beziehungsweise Alkylesters desselben vor ihr stehenden Aminosäure oder einem an seinem endständigen Stickstoffatom und gegebenenfalls an der Seitenfunktion geschützten und an der Carboxylgruppe aktivierten in der Aminosäuresequenz des herzustellenden Peptides beziehungsweise Alkylesters desselben vor ihr stehende Aminosäuren aufweisenden Peptidfragmentderivat acyliert wird und gegebenenfalls in [einer] nächsten Stufe(n) mit dem erhaltenen am endständigen Stickstoffatom geschützten Peptidzwischenprodukt und dem beziehungsweise den durch [eine] etwaige weitere derartige Acylierung(en) erhaltenen am endständigen Stickstoffatom geschützten weiteren Peptidzwischenprodukt(en) nach in an sich bekannter Weise erfolgendem Entfernen der Schutzgruppe des zu acylierenden endständigen Stickstoffatomes eine weitere beziehungsweise weitere Acylierung(en) mit beziehungsweise jeweils mit einem an ihrem beziehungsweise seinem endständigen Stickstoffatom geschützten und an der Carboxylgruppe aktivierten Derivat der in der Aminosäuresequenz des herzustellenden Peptides beziehungsweise Salzes, Komplexes, Amides beziehungsweise Alkylesters desselben vor ihr stehenden Aminosäure oder einem an seinem endständigen Stickstoffatom und gegebenenfalls an der Seitenfunktion geschützten und an der Carboxylgruppe aktivierten in der Aminosäuresequenz des herzustellenden Peptides beziehungsweise Salzes, Komplexes, Amides beziehungsweise Alkylesters desselben vor ihr stehende Aminosäuren aufweisenden Peptidfragmentderivat vorgenommen wird, wobeil so viele Acylierungen durchgeführt werden, wie es zum Erreichen der gewünschten Aminosäuresequenz erforderlich ist, und gegebenenfalls danach in an sich bekannter Weise vom erhaltenen geschützten Peptid die Schutzgruppe des endständigen Stickstoffatomes und die etwaige(n) andere(n) Schutzgruppe(n) entfernt wird beziehungsweise werden und gegebenenfalls in an sich bekannter Weise das erhaltene geschützte oder von der beziehungsweise den Schutzgruppe(n) befreite Peptid der allgemeinen Formeln I, II, III, IV, V, VI, VII, VIII, IX, X, XI, XII, XIII, XIV beziehungsweise XV in ein Säureadditionssalz oder einen Komplex überführt wird beziehungsweise gegebenenfalls das erhaltene Säureadditionssalz des Peptides der allgemeinen Formeln I, II, III, IV, V, VI, VII, VIII, IX, XI, XII, XIII, XIV beziehungsweise XV in das Peptid der allgemeinen Formeln I, II, III, IV, V, VI, VII, VIII, IX, X, XI, XII, XIII, XIV beziehungsweise XV oder in ein anderes Säureadditionssalz überführt wird.

Vorteilhaft werden die erfindungsgemäßen Peptide in Lösung nach dem Prinzip des schrittweisen Aufbaues mit Hilfe bekannter peptidchemischer Verfahrensweisen hergestellt. Bei dieser Synthese kommt vor allem eine Schutzgruppenkombination, welche das selektive Entfernen und das möglichst in 1 Stufe erfolgende Entfernen der Schutzgruppe(n) vom geschützten Peptid ermöglicht, zum Einsatz.

Zur Ausbildung der Peptidbindung ist es bevorzugt, zum Acylieren als aktiviertes Derivat einen aktiven Ester oder ein gemischtes Anhydrid der acylierenden Aminosäure beziehungsweise des acylierenden Peptidfragmentes zu verwenden, wobei es besonders bevorzugt ist, das bei eigenen

# 0 067 425

früheren Forschungen ausgearbeitete Verfahren über den Pentafluorphenylester (Kisfaludy und Mitarbeiter : Tetrahedron Letters [1974], 1 785 : ungarische Patentschrift 168 431) anzuwenden.

Eine besonders bevorzugte Ausführungsform des erfindungsgemäßen Verfahrens ist an Hand der Synthese des Tetrapeptides L-Arginyl-L-lysyl-L-asparagyl-L-valin wie folgt. L-Valin-p-nitrobenzylester wird mit [N-(tert.-Butoxycarbonyl)]-L-asparaginsäure-β-benzylester-α-pentafluorphenylester acyliert, vom erhaltenen geschützten Dipeptid [N-(tert.-Butoxycarbonyl)]-L-asparagyl-[β-(benzylester)]-L-valin-p-nitrobenzylester wird die tert.-Butoxycarbonylgruppe selektiv entfernt und das freie (von dieser befreite Dipeptid L-Asparagyl-[β-(benzylester)]-L-valin-p-nitrobenzylester wird mit [Nα-(tert.-Butoxycarbonyl)-Nε-(benzyloxycarbonyl)]-L-lysinpentafluorphenylester acyliert. Durch Behandeln des erhaltenen geschützten Tripeptides [Nα-(tert.-Butoxycarbonyl)-Nε-(benzyloxycarbonyl)]-L-lysyl-L-asparagyl-[β-(benzylester)]-L-valin-p-nitrobenzylester mit Trifluoressigsäure wird das am N-Ende freie Tripeptid erhalten, welches mit Nα-[(Benzyloxycarbonyl)-NG-(nitro)]-L-argininpentafluorphenylester acyliert wird. Sämtliche Schutzgruppen des auf diese Weise erhaltenen geschützten Tetrapeptides [Nα-(Benzyloxycarbonyl)-NG-(nitro)]-L-arginyl-[Nε-(benzyloxycarbonyl)]-L-lysyl-L-asparagyl-[β-(benzylester)]-L-valin-p-nitrobenzylester können durch katalytisches Hydrieren in einer einzigen Stufe entfernt werden.

In den meisten Fällen ist eine weitere Reinigung der erhaltenen Peptide nicht erforderlich. Erforderlichenfalls können die freien Peptide durch Säulenchromatographie an Silicagel gereinigt werden. In diesem Fall wird das Endprodukt durch einfaches Lyophilisieren oder Eindampfen erhalten. Gegebenenfalls werden die erhaltenen Peptide wie bereits erwähnt in ihre Salze oder Komplexe überführt.

Ferner sind erfindungsgemäß Arzneimittel, welche 1 oder mehr erfindungsgemäße Verbindung(en) als Wirkstoff(e), zweckmäßigerweise zusammen mit 1 oder mehr üblichen pharmazeutischen Konfektionierungsmittel(n), enthalten, vorgesehen.

Die erfindungsgemäßen Verbindungen haben nämlich wie bereits erwähnt wertvolle pharmakologische Wirkungen, insbesondere die Immunregulation beeinflussende Wirkungen, wie Wirkungen auf die Aktivität der Lymphozyten, auf die Antikörpererzeugung und auf die spezifische Abwehrstoffe erzeugenden Zellen.

Zur biologischen Untersuchung der erfindungsgemäßen Peptide wurden folgende Untersuchungsverfahren herangezogen.

## A. In vitro

Die Untersuchung auf aktive E-Rosetten wurde nach der modifizierten Verfahrensweise von Wybran und Mitarbeitern (Wybran und Mitarbeiter : New Engl. J. Med. *292* [1975], 475 mit Lymphozyten von zum Teil gesunden und zum Teil autoimmunen kranken Menschen (rheumatoide Arthritis und systemischer Lupus erythematosus) vorgenommen. Zu 50 µl Lymphozytenzellsuspension wurden jeweils 100 µl der auf eine Konzentration von $10^{-3}$ bis $10^{-11}$ Mol/l verdünnten zu untersuchenden Substanz zugegeben. Das Gemisch wurde 60 Minuten lang bei 37 °C in 5 Vol.-% Kohlendioxyd enthaltender Luft bebrütet und dann mit 50 µl einer 1 gew.-%-igen (auf das Volumen bezogen) Suspension von roten Blutkörperchen (vom Schaf) versetzt. Dann wurde 10 Minuten lang mit einer Drehzahl von 1 000 Minuten$^{-1}$ zentrifugiert. Danach wurden die Gläschen auf eine modifizierte Gallenkampf-Schüttelmaschine gesetzt und 30 Sekunden lang geschüttelt (Schüttelfrequenz : 65 Minuten$^{-1}$, Hubhöhe 8 cm). Zwecks Fixierung der Rosetten wurden jedem Röhrchen 50 µl 0,1 gew.-%-iger (auf das Volumen bezogen) Glutaraldehyd zugesetzt. Nach 3 Minuten wurden unter dem Mikroskop die mehr als 3 der vom Schaf stammenden roten Blutkörperchen bindenden Lymphozyten gezählt, wobei insgesamt $4 \times 100$ Zellen ausgewertet wurden. Die abgetrennten Lymphozyten enthielten als Verunreinigung Makrophagen und polymorphkernige (polymorphonukleare) Zellen (höchstens 10 %). Die Anzahl der Rosetten bildenden Zellen wurde in jedem Fall um diesen Wert korrigiert. Die Ergebnisse sind in den folgenden Tabellen 1 und 2 zusammengestellt.

## Tabelle 1

**E-Rosettenbildende Aktivität der Lymphozyten von an rheumatoider Arthritis Leidenden**
**(in Prozenten der aktiven Lymphozyten ausgedrückt ; n [Zahl der Parallelversuche] = 4)**

| Peptid | | Aktivität bei der Peptidkonzentration (in Mol/l), ausgedrückt als ihr negativer dekadischer Logarithmus, | | | | | |
|---|---|---|---|---|---|---|---|
| Bei-spiel Nr. | Bezeichnung | 0 | 11 | 9 | 7 | 5 | 3 |
| 4 | L-Arginyl-L-lysyl-L-asparagyl-L-valin | 29,4 | 32,6 | 36,8 | 34,4 | 31,5 | 36,2 |
| 19 | L-Arginyl-L-lysyl-L-asparaginsäure **) | 26,4 | 28,4 | 30,7 | 28,8 | 28,6 | 27,2 |
| 20 | L-Arginyl-L-lysyl-L-asparagyl-L-valinamid | 26,4 | 28,2 | 34,8 | 26,8 | 29,8 | 31,5 |
| 21 | L-Lysyl-L-arginyl-L-asparagyl-L-valin | 20,8 | 26,6 | 25,7 | 21,2 | 25,8 | 23,6 |
| 22 | L-Arginyl-L-lysyl-L-asparagyl-L-valin-methylester | 26,4 | 26,5 | 27,9 | 32,4 | 30,8 | 31,2 |
| 23 | L-Pyroglutamyl-L-arginyl-L-lysyl-L-asparagyl-L-valin | 23,7 | 20,4 | 19,4 | 21,4 | 23,2 | 22,8 |

Fortsetzung der Tabelle 1

| Peptid | | Aktivität bei der Peptidkonzentration (in Mol/l), ausgedrückt als ihr negativer dekadischer Logarithmus, | | | | | |
|---|---|---|---|---|---|---|---|
| Bei-spiel Nr. | Bezeichnung | 0 | 11 | 9 | 7 | 5 | 3 |
| 24 | L-Pyroglutamyl-L-arginyl-L-lysyl-L-aspa-ragin **) | 23,7 | 18,4 | 19,7 | 19,5 | 18,7 | 20,6 |
| 25 | L-Arginyl-L-lysyl-L-asparaginyl-L-valin | 27,7 | 24,3 | 23,7 | 24,6 | 21,9 | 22,3 |
| 29 | L-Arginyl-L-lysyl-L-aminosuccinyl-L-valin | 20,8 | 25,0 | 28,6 | 24,3 | 22,5 | 23,6 |
| 27 | L-Arginyl-L-alanyl-L-asparagyl-L-valin | 23,7 | 22,5 | 20,7 | 19,8 | 20,2 | 21,8 |
| 28 | L-Arginyl-L-lysyl-L-alanyl-L-valin | 20,8 | 23,1 | 25,5 | 21,7 | 23,4 | 20,3 |
| 26 | L-Alanyl-L-lysyl-L-asparagyl-L-valin | 23,7 | 20,3 | 23,9 | 22,5 | 22,8 | 25,3 |
| 30 | L-Arginyl-L-lysyl-L-asparagyl-L-alanin | 26,4 | 27,9 | 25,3 | 25,8 | 24,5 | 28,6 |
| 31 | L-Arginyl-L-lysyl-L-asparagyl-L-isoleucin | 27,7 | 24,5 | 28,7 | 23,2 | 25,4 | 25,5 |

0 067 425

Fortsetzung der Tabelle 1

| Peptid | | Aktivität bei der Peptidkonzentration (in Mol/l), ausgedrückt als ihr negativer dekadischer Logarithmus, | | | | | |
|---|---|---|---|---|---|---|---|
| Bei-spiel Nr. | Bezeichnung | 0 | 11 | 9 | 7 | 5 | 3 |
| 32 | L-Arginyl-L-asparagyl-L-lysyl-L-valin | 27,7 | 27,5 | 27,3 | 26,4 | 26,2 | 25,4 |
| 33 | L-Pyroglutamyl-L-arginyl-L-lysyl-L-asparagyl-L-valyl-L-tyrosin | 20,8 | 24,9 | 26,6 | 22,6 | 22,7 | 24,1 |
| Vergleichs-substanz | L-Arginyl-L-lysyl-L-asparagyl-L-valyl--L-tyrosin (TP5) | 23,6 | 26,7 | 26,8 | 29,9 | 26,7 | – |

**) $p < 0.05$ F Prüfversuch [mit der Einparameter-Varianzanalyse, bezogen auf L-Arginyl-L-lysyl-L-asparagyl-L-valyl-L-tyrosin (TP5)]

$p < 0.05$ mit dem Student-t-Prüfversuch

## Tabelle 2

E-Rosettenbildende Aktivität der Lymphozyten von Gesunden
(in Prozenten der aktiven Lymphozyten ausgedrückt ; n [Zahl der Parallelversuche] = 4)

| Peptid | | Aktivität bei der Peptidkonzentration (in Mol/l), ausgedrückt als ihr negativer dekadischer Logarithmus, | | | | | |
| Bei-spiel Nr. | Bezeichnung | 0 | 11 | 9 | 7 | 5 | 3 |
|---|---|---|---|---|---|---|---|
| 4 | L-Arginyl-L-lysyl-L-asparagyl-L-valin **) | 33,8 | 33,7 | 43,0 | 38,5 | 39,1 | 41,3 |
| 19 | L-Arginyl-L-lysyl-L-asparaginsäure | 30,3 | 35,9 | 39,3 | 41,7 | 34,1 | 29,9 |
| 20 | L-Arginyl-L-lysyl-L-asparagyl-L-valin-amid | 30,3 | 38,1 | 26,6 | 35,1 | 33,8 | 29,0 |
| 30 | L-Arginyl-L-lysyl-L-asparagyl-L-alanin | 35,3 | 40,5 | 36,4 | 40,8 | 39,6 | 41,2 |
| 26 | L-Alanyl-L-lysyl-L-asparagyl-L-valin | 35,3 | 36,2 | 39,1 | 47,5 | 34,9 | 43,4 |
| 22 | L-Arginyl-L-lysyl-L-asparagyl-L-valin-methylester | 29,7 | 23,8 | 24,8 | 29,4 | 27,2 | 26,8 |
| 32 | L-Arginyl-L-asparagyl-L-lysyl-L-valin ***) | 32,1 | 36,4 | 40,4 | 37,7 | 37,5 | 34,2 |

Fortsetzung der Tabelle 2

| Peptid | | Aktivität bei der Peptidkonzentration (in Mol/l), ausgedrückt als ihr negativer dekadischer Logarithmus, | | | | | |
|---|---|---|---|---|---|---|---|
| Bei-spiel Nr. | Bezeichnung | 0 | 11 | 9 | 7 | 5 | 3 |
| 24 | L-Pyroglutamyl-L-arginyl-L-lysyl-L-as-paragin | 30,3 | 38,1 | 26,6 | 35,1 | 33,8 | 29,1 |
| 28 | L-Arginyl-L-lysyl-L-alanyl-L-valin | 34,7 | 30,9 | 34,5 | 35,8 | 30,5 | 33,3 |
| 27 | L-Arginyl-L-alanyl-L-asparagyl-L-valin | 34,7 | 36,0 | 38,2 | 32,4 | 33,4 | 32,5 |
| 21 | L-Lysyl-L-arginyl-L-asparagyl-L-valin | 28,9 | 32,9 | 29,1 | 30,0 | 25,6 | 31,5 |
| 29 | L-Arginyl-L-lysyl-L-aminosuccinyl-L--valin | 29,7 | 31,5 | 32,3 | 29,0 | 29,7 | 29,3 |
| 34 | L-Arginyl-L-lysyl-L-glutamyl-L-valin **) | 31,9 | 33,0 | 35,4 | 36,2 | 32,9 | 33,2 |
| 25 | L-Arginyl-L-lysyl-L-asparaginyl-L--valin **) | 32,1 | 36,7 | 35,4 | - | 39,1 | 35,5 |

Fortsetzung der Tabelle 2

| Peptid | | Aktivität bei der Peptidkonzentration (in Mol/l), ausgedrückt als ihr negativer dekadischer Logarithmus, | | | | | |
| Bei-spiel Nr. | Bezeichnung | 0 | 11 | 9 | 7 | 5 | 3 |
|---|---|---|---|---|---|---|---|
| 31 | L-Arginyl-L-lysyl-L-asparagyl-L-iso-leucin **) | 33,2 | 30,7 | 35,0 | 34,9 | 32,0 | 35,5 |
| 33 | L-Pyroglutamyl-L-arginyl-L-lysyl-L-asparagyl-L-valyl-L-tyrosin | 24,1 | 29,9 | 25,5 | 29,4 | 22,5 | 29,9 |
| Ver-gleichs-substanz | L-Arginyl-L-lysyl-L-asparagyl-L-valyl--L-tyrosin (TP5) | 27,8 | 25,2 | 27,1 | 24,4 | 23,7 | 29,4 |

**) $P < 0,05$ F Prüfversuch [mit der Einparameter-Varianzanalyse, bezogen auf L-Arginyl-L-lysyl-L-asparagyl-L-valyl-L-tyrosin (TP5)]

***) $P < 0,01$ F Prüfversuch [mit der Einparameter-Varianzanalyse, bezogen auf L-Arginyl-L-lysyl-L-asparagyl-L-valyl-L-tyrosin (TP5)]; $P < 0,05$ Student t signifikante Wirkung

Aus der obigen Tabelle 1 geht hervor, daß die E-Rosettenbildungsaktivität der Lymphozyten von an rheumatoider Arthritis Leidenden durch das Tetrapeptid L-Arginyl-L-lysyl-L-asparagyl-L-valin (Beispiel 4) in einer Konzentration von $10^{-9}$ Mol/l signifikant angeregt beziehungsweise stimuliert wird. Ebenfalls in einer Konzentration von $10^{-9}$ Mol/l wirken das Amid dieses Tetrapeptides, nämlich L-Arginyl-L-lysyl-L-asparagyl-L-valinamid (Beispiel 20) sowie ferner das Tripeptid L-Arginyl-L-lysyl-L-asparaginsäure (Beispiel 19), das Tetrapeptid L-Lysyl-L-arginyl-L-asparagyl-L-valin (Beispiel 21), das Tetrapeptid L-Arginyl-L-lysyl-L-aminosuccinyl-L-valin (Beispiel 29) und das Hexapeptid L-Pyroglutamyl-L-arginyl-L-lysyl-L-asparagyl-L-valyl-L-tyrosin (Beispiel 33 anregend beziehungsweise stimulierend. Das Tetrapeptid L-Lysyl-L-arginyl-L-asparagyl-L-valin (Beispiel 21) wirkt noch in einer Konzentration von $10^{-11}$ Mol/l und der Tetrapeptidester L-Arginyl-L-lysyl-L-asparagyl-L-valinmethylester (Beispiel 22) in einer Konzentration von $10^{-7}$ Mol/l anregend beziehungsweise stimulierend. Eine signifikante Hemmwirkung zeigten die mit Pyroglutaminsäure beginnenden Penta- und Tetrapeptid L-Pyroglutamyl-L-arginyl-L-lysyl-L-asparagyl-L-valin (Beispiel 23) beziehungsweise L-Pyroglutamyl-L-arginyl-L-lysyl-L-asparagin (Beispiel 24) in Konzentrationen von $10^{-9}$ beziehungsweise $10^{-11}$ Mol/l und das Asparagin aufweisende Tetrapeptid L-Arginyl-L-lysyl-L-asparaginyl-L-valin (Beispiel 25) in einer Konzentration von $10^{-5}$ Mol/l.

Die Daten der obigen Tabelle 2 zeigen, daß die E-Rosettenbildungsaktivität der Lymphozyten von gesunden Personen durch das Tetrapeptid L-Arginyl-L-lysyl-L-asparagyl-L-valin Beispiel 4) in einer Konzentration von $10^{-9}$ Mol/l, das Tripeptid L-Arginyl-L-lysyl-L-asparaginsäure (Beispiel 19) und das Tetrapeptid L-Arginyl-L-lysyl-L-glutamyl-L-valin (Beispiel 34) in einer Konzentration von $10^{-7}$ Mol/l, das Tetrapeptid L-Alanyl-L-lysyl-L-asparagyl-L-valin (Beispiel 26) in einer Konzentration von $10^{-7}$ Mol/l und das Tetrapeptid L-Arginyl-L-asparagyl-L-lysyl-L-valin (Beispiel 32) in einer Konzentration von $10^{-9}$ Mol/l signifikant angeregt beziehungsweise stimuliert wird. Ebenfalls anregend beziehungsweise stimulierend wirken das Amid des Tetrapeptides L-Arginyl-L-lysyl-L-asparagyl-L-valin (Beispiel 4), das L-Arginyl-L-lysyl-L-asparagyl-L-valinamid (Beispiel 20), das Tetrapeptid L-Arginyl-L-lysyl-L-asparagyl-L-alanin (Beispiel 30) und das Tetrapeptid L-Pyroglutamyl-L-arginyl-L-lysyl-L-asparagin (Beispiel 24) sowie das Hexapeptid L-Pyroglutamyl-L-arginyl-L-lysyl-L-asparagyl-L-valyl-L-tyrosin (Beispiel 33) in einer Konzentration von $10^{-11}$ Mol/l. Ferner wirkt das Tetrapeptid L-Arginyl-L-lysyl-L-asparaginyl-L-valin (Beispiel 25) in einer Konzentration von $10^{-5}$ Mol/l anregend beziehungsweise stimulierend. Der Merthylester des Tetrapeptides L-Arginyl-L-lysyl-L-asparagyl-L-valin (Beispiel 4), der L-Arginyl-L-lysyl-L-asparagyl-L-valinmethylester (Beispiel 22) erwies sich in diesem Versuch in einer Konzentration von $10^{-11}$ Mol/l als hemmend

Ferner geht aus den obigen Tabellen 1 und 2 die Überlegenheit der erfindungsgemäßen Verbindungen gegenüber der Vergleichssubstanz L-Arginyl-L-lysyl-L-asparagyl-L-valyl-L-tyrosin (TP5) hervor. •

B. In vivo

a) Zur Untersuchung der auf die Antikörpererzeugung ausgeübten Wirkung wurde die Verfahrensweise von Ceglowski gewählt (Ceglowski : Ann. N.Y. Acad. Sci. 249 [1975], 343). Neugeborenen Wistar-Ratten wurde spätestens in der 12-ten Stunde nach ihrer Geburt die zu untersuchende Substanz in Form von 25 μl Flüssigkeit der Konzentration von $4.10^{-3}$ bis $4.10^{-7}$ Mol/l intraperitoneal verabreicht (je Dosis je 9 Tiere). Im Alter von 14 Tagen wurden die Tiere durch intraperitoneale Verabreichung von je 0,5 cm³ einer 5 %-igen Suspension von roten Blutkörperchen von Schafen immunisiert, und am 7-ten Tag nach der Immunisierung wurden die Tiere durch Abtrennen des Kopfes ausbluten gelassen. Das Blut von je 3 Tieren wurde vereinigt und 10 Minuten lang mit einer Drehzahl von 3 000 Minute$^{-1}$ zentrifugiert und im auf diese Weise gewonnenen Serum wurde der Antikörpertiter nach der Verfahrensweise von Takátsy (Takátsy : Acta Microbiol. Acad. Sci. Hung. 3 [1955], 191) bestimmt. Die Ergebnisse wurden als Agglutinationstiter ausgedrückt, wobei als Titer diejenige größte Serumverdünnung angesehen wurde, bei der die Agglutination noch wahrnehmbar ist. Die Ergebnisse sind in der unten folgenden Tabelle 3 zusammengestellt.

b) Die Anzahl der spezifische Antistoffe erzeugenden Zellen wurde nach der Verfahrensweise von Canningham bestimmt (Handbook of Experimental Immunology, redigiert von D.M. Weir, Band 2, Seite 285, Blackwell, Oxford-London [1978]). Das Wesen der Verfahrensweise besteht darin, daß aus den Milzzellen der immunisierten Tiere, aus einer Suspension von roten Blutkörperchen von Schafen und einem Komplement eine homogene Suspension bereitet und in eine zur Ausbildung von Monozellschichten geeignete Kammer eingebracht wird. Um die Antistoffe erzeugenden Zellen herum bildet sich ein lytischer Hof aus und die Anzahl dieser Höfe (Flecken) ist identisch mit der Anzahl der spezifischen Antistoff erzeugenden Zellen (plaquebildenden Zellen) [plaque forming cells, PFC].

Den Versuchstieren wurden spätestens in der 12-ten Stunde nach ihrer Geburt 1-mal die zu untersuchenden Substanzen intraperitoneal verabreicht. Jede Substanz wurde wie bei der Verfahrensweise a) in 3 Dosen untersucht. Am 7-ten Tag nach der Immunisierung wurde die Fähigkeit der aus den Tieren präparierten Milzzellen zur Plaquebildung untersucht. In der folgenden Tabelle 4 ist der Quotient aus der Anzahl der Plaques bei den behandelten Tieren und der Anzahl der Plaques bei den unbehandelten Blindversuchs- beziehungsweise Kontrolltieren angegeben.

## Tabelle 3

### Die auf die Antikörpererzeugung ausgeübte Wirkung in vivo, ausgedrückt als Agglutinationstiter
### (Antikörpertiter = A ± S.D.)

| Peptid | | Agglutinationstiter bei der Konzentration (in Mol/l) | | | | | | |
|---|---|---|---|---|---|---|---|---|
| Bei-spiel Nr. | Bezeichnung | $4 \cdot 10^{-3}$ | $4 \cdot 10^{-4}$ | $4 \cdot 10^{-5}$ | $4 \cdot 10^{-6}$ | $4 \cdot 10^{-7}$ | $4 \cdot 10^{-8}$ | 0 [Blindversuch] |
| 30 | L-Arginyl-L-ly-syl-L-asparagyl--L-alanin **) | $10,5 \pm 1,5$ | – | $11,5 \pm 0,9$ | – | $12,0 \pm 0$ | – | $9,3 \pm 1,5$ |
| 19 | L-Arginyl-L-ly-syl-L-asparagin-säure **) | – | $6,0 \pm 0$ | – | $7,3 \pm 0,6$ | – | $6,0 \pm 1,2$ | $5,2 \pm 1,3$ |
| 20 | L-Arginyl-L-ly-syl-L-asparagyl--L-valinamid. | – | $5,2 \pm 2,2$ | – | $6,2 \pm 2,0$ | – | $6,8 \pm 0,8$ | $5,2 \pm 1,3$ |

Fortsetzung der Tabelle 3

| Peptid | | Agglutinationstiter bei der Konzentration (in Mol/l) | | | | | | |
|---|---|---|---|---|---|---|---|---|
| Bei-spiel Nr. | Bezeichnung | $4 \cdot 10^{-3}$ | $4 \cdot 10^{-4}$ | $4 \cdot 10^{-5}$ | $4 \cdot 10^{-6}$ | $4 \cdot 10^{-7}$ | $4 \cdot 10^{-8}$ | 0 [Blindversuch] |
| 33 | L-Pyroglutamyl-L--arginyl-L-lysyl--L-asparagyl-L--valyl-L-tyrosin **) | $3,0 \pm 1,0^{O)}$ | – | $5,2 \pm 0,8^{O)}$ | – | – | – | $5,2 \pm 1,3$ |
| 23 | L-Pyroglutamyl-L--arginyl-L-lysyl--L-asparagyl-L-valin | $9,0 \pm 2,6$ | – | $8,3 \pm 0,6$ | – | $7,2 \pm 1,3$ | – | $9,3 \pm 1,5$ |

0 067 425

Fortsetzung der Tabelle 3

| Peptid | | Agglutinationstiter bei der Konzentration (in Mol/l) | | | | | | |
|---|---|---|---|---|---|---|---|---|
| Bei- spiel Nr. | Bezeichnung | $4 \cdot 10^{-3}$ | $4 \cdot 10^{-4}$ | $4 \cdot 10^{-5}$ | $4 \cdot 10^{-6}$ | $4 \cdot 10^{-7}$ | $4 \cdot 10^{-8}$ | 0 [Blindversuch] |
| Ver- gleichs- substanz | L-Arginyl-L-lysyl- -L-asparagyl-L- -valyl-L-tyrosin | $12,0 \pm 0$ | $8,7 \pm 2,3$ | $10,7 \pm 2,3$ | $9,0 \pm 1,7$ | – | – | $9,3 \pm 1,5$ |

**) P < 0,05 mit dem Student t Prüfversuch
0) Die Konzentration der untersuchten Substanz betrug nur die Hälfte des in der Kopfspalte angegebenen Wertes

0 067 425

Tabelle 4

Die auf die plaquebildenden Milzzellen ausgeübte Wirkung

| Peptid | | Dosis in Millimol | Quotient | Anzahl der Plaques bei den behandelten Tieren / Anzahl der Plaques bei den Blindversuchstieren |
|---|---|---|---|---|
| Bei-spiel Nr. | Bezeichnung | | | |
| 19 | L-Arginyl-L-lysyl-L-aspa-raginsäure | $1,0 \cdot 10^{-4}$ | | 0,7 |
| | | $1,0 \cdot 10^{-6}$ | | 2,1 |
| | | $1,0 \cdot 10^{-8}$ | | 1,8 |
| 20 | L-Arginyl-L-lysyl-L-aspa-ragyl-L-valinamid | $1,0 \cdot 10^{-4}$ | | 0,3 |
| | | $1,0 \cdot 10^{-6}$ | | 0,52 |
| | | $1,0 \cdot 10^{-8}$ | | 2,7 |

Fortsetzung der Tabelle 4

Die auf die plaquebildenden Milzzellen ausgeübte Wirkung

| Peptid | | Dosis in Millimol | Quotient | Anzahl der Plaques bei den behandelten Tieren / Anzahl der Plaques bei den Blindversuchstieren |
|---|---|---|---|---|
| Bei-spiel Nr. | Bezeichnung | | | |
| 4 | L-Arginyl-L-lysyl-L-aspa-ragyl-L-valin | $2,5 \cdot 10^{-3}$ | | 0,34 |
| | | $2,5 \cdot 10^{-4}$ | | 0,8 |
| 30 | L-Arginyl-L-lysyl-L-aspa-ragyl-L-alanin | $1,0 \cdot 10^{-4}$ | | 4,9 |
| | | $1,0 \cdot 10^{-6}$ | | 1,9 |
| | | $1,0 \cdot 10^{-8}$ | | 3,0 |
| 26 | L-Alanyl-L-lysyl-L-aspa-ragyl-L-valin | $1,0 \cdot 10^{-4}$ | | 0,04 |
| | | $1,0 \cdot 10^{-6}$ | | 0,01 |

0 067 425

Gemäß den auf die Antikörpererzeugung ausgeübte und durch den Agglutinationstiter ausgedrückte Wirkung in vivo betreffenden Daten der obigen Tabelle 3 zeigen eine signifikant anregende beziehungsweise stimulierende Wirkung das Tripeptid L-Arginyl-L-lysyl-L-asparaginsäure (Beispiel 19) in einer Konzentration von $4 \cdot 10^{-6}$ Mol/l, das Tetrapeptid L-Arginyl-L-lysyl-L-asparagyl-L-alanin (Beispiel 30) in einer Konzentration von $4 \cdot 10^{-7}$ Mol/l und das Tetrapeptidamid L-Arginyl-L-lysyl-L-asparagyl-L-valinamid (Beispiel 20) in einer Konzentration von $4 \cdot 10^{-8}$ Mol/l. Eine hemmende Wirkung zeigten auch in diesem Versuch die mit Pyroglutaminsäure beginnenden Peptide L-Pyroglutamyl-L-arginyl-L-lysyl-L-asparagyl-L-valin (Beispiel 23) in einer Konzentration von $4 \cdot 10^{-7}$ Mol/l und L-Pyroglutamyl-L-arginyl-L-lysyl-L-asparagyl-L-valyl-L-tyrosin (Beispiel 33) in einer Konzentration von $2 \cdot 10^{-3}$ Mol/l.

Aus den Werten der die auf die plaquebildenden Milzzellen in vivo ausgeübte Wirkung betreffenden obigen Tabelle 4 geht hervor, daß das Tripeptid L-Arginyl-L-lysyl-L-asparaginsäure (Beispiel 19) und das Tetrapeptid-amid L-Arginyl-L-lysyl-L-asparagyl-L-valinamid (Beispiel 20) in kleineren Dosen eine Erhöhung der Anzahl der Plaques gegenüber denen bei den Blindversuchstieren auf etwa das Doppelte bewirkten, während das Tetrapeptid L-Arginyl-L-lysyl-L-asparagyl-L-alanin (Beispiel 30) in jeder Dosis eine beträchtliche Erhöhung der Plaques herbeiführte. In den untersuchten Dosen hatte das Tetrapeptid L-Alanyl-L-lysyl-L-asparagyl-L-valin (Beispiel 26) und in der Dosis von $2{,}5 \cdot 10^{-3}$ das Tetrapeptid L-Arginyl-L-lysyl-L-asparagyl-L-valin (Beispiel 4) eine ausgeprägte Hemmwirkung.

Gemäß der obigen Tabelle 1 haben die erfindungsgemäßen Peptide bei Konzentrationen ab $10^{-11}$ Mol/l = $10^{-14}$ Mol/ml, welchen mit einem Molekulargewicht von 1 000 gerechnet $10^{-11}$ g/ml = 0,01 ng/ml entsprechen, die maximale Wirkung. Diese Mindestkonzentration der maximalen Wirkung ist wesentlich geringer als die in der britischen Patentschrift 1 565 032 angegebene von ab etwa 100 mg/ml.

Andererseits geht aus der obigen Tabelle 4 hervor, daß mit den erfindungsgemäßen Peptiden die Wirkung bei Dosen von $10^{-4}$ bis $10^{-8}$ Millimol/Tier = $10^{-7}$ bis $10^{-11}$ Mol/Tier erzielt wurde, was bei Zugrundelegung eines Molekulargewichtes von 1 000 $10^{-4}$ bis $10^{-8}$ g/Tier = 10 ng bis 100 μg/Tier entspricht. Diese Dosen sind wesentlich geringer als die in der britischen Patentschrift 1 565 032 angegebenen von 10 bis 100 μg/Tier.

Die durch die Erfindung erzielten überraschenden Wirkungen sind zusammengefaßt wie folgt :

a) Die Auffassung, daß L-Arginyl-L-lysyl-L-asparagyl-L-valyl-L-tyrosin [TP5] das kleinste Fragment, welches eine T-Zellendifferenzierungswirkung hat, ist, wurde umgestoßen.

b) Es wurde festgestellt, daß, wenn die Kettenverkürzung von L-Arginyl-L-lysyl-L-asparagyl-L-valyl-L-tyrosin [TP5] am C-ständigen Ende begonnen wird, auch die kürzeren Fragmente eine bedeutende und vielfach bedeutend höhere Wirksamkeit zeigen als das L-Arginyl-L-lysyl-L-asparagyl-L-valyl-L-tyrosin [TP5].

c) Es wurden auch Peptide, deren Wirkung umgekehrt ist, das heißt, daß sie die T-Zellendifferenzierung hemmen, festgestellt. Peptide mit solcher Wirkung sind in keiner der Vorveröffentlichungen erwähnt.

d) Auch die erfindungsgemäßen Tetrapeptide [TP4] und Tripeptide [TP3] sind stabil, ihre Imidbildung ist gehemmt beziehungsweise unmöglich, sie können einfach und wirtschaftlich hergestellt werden und auch ihre Überführung in Zubereitungen ist mit keinen Problemen verbunden.

Die erfindungsgemäßen Peptide einschließlich ihrer Salze, Komplexe, Amide und Ester können in Form von üblichen Arzneimittelpräparaten vorliegen und in der Therapie angewandt werden. Diese Arzneimittelpräparate enthalten die erfindungsgemäßen Verbindungen zusammen mit zur enteralen oder parenteralen Verabreichung geeigneten anorganischen oder organischen Träger- und/oder Hilfsstoffen. Sie können in Form von festen Lyophilisaten, welche als Trägerstoffe pharmazeutisch brauchbare und mit den erfindungsgemäßen Peptiden nicht reagierende Substanzen, zum Beispiel Kohlehydrate, enthalten, sowie ferner in Form von verdünnten oder konzentrierten Suspensionen und Emulsionen und weiterhin Tabletten und Injektionslösungen vorliegen. Die Zubereitung dieser Arzneimittelpräparateformen kann nach üblichen pharmazeutischen Verfahrensweisen erfolgen.

Die Erfindung wird an Hand der folgenden Beispiele näher erläutert. Die in diesem Text verwendeten Abkürzungen sind die im chemischen Fachschrifttum üblichen Abkürzungen (J. Biol. Chem. *247* [1972], 977). Weitere Abkürzungen sind : Z = Benzyloxycarbonylgruppe, Boc = tert.-Butoxycarbonylgruppe, O$^t$Bu = tert.-Butoxygruppe, OPfp = Pentafluorphenoxygruppe, Asu = L-Aminosuccinylgruppe, OMe = Methoxygruppe, OBzl = Benzyloxygruppe, ONB = 4-Nitrobenzyloxygruppe und Su = $N_{succ}$-oxysuccinimidgruppe.

Zur Bestimmung der Schmelzpunkte wurde ein Schmelzpunktmeßgerät nach Dr. Tottoli (Büchi, Schweiz) verwendet. Die Dünnschichtchromatogramme wurden an vorgefertigten Silicagelplatten (DC-Fertigplatten®, Merck) mit folgenden Lösungsmittelgemischen aufgenommen :

Lösungsmittelgemisch Volumverhältnis der Lösungsmittel beziehungsweise des vor der eckigen Klammer stehenden Lösungsmittels zum in den eckigen Klammern stehenden Lösungsmittelgemisch

1. Äthylacetat : [Pyridin/Essigsäure/Wasser im Volumverhältnis von 20 : 6 : 11]   = 95 : 5
2. Äthylacetat : [Pyridin/Essigsäure/Wasser im Volumverhältnis von 20 : 6 : 11]   = 9 : 1
3. Äthylacetat : [Pyridin/Essigsäure/Wasser im Volumverhältnis von 20 : 6 : 11]   = 4 : 1
4. Äthylacetat : [Pyridin/Essigsäure/Wasser im Volumverhältnis von 20 : 6 : 11]   = 3 : 2
5. n-Butanol : [Pyridin/Essigsäure/Wasser im Volumverhältnis von 20 : 6 : 11]   = 3 : 7

| | |
|---|---|
| 6. Chloroform : Methanol | = 9 : 1 |
| 7. n-Butanol : Essigsäure : Wasser | = 1 : 1 : 1 |
| 8. n-Butanol : Essigsäure : Wasser | = 4 : 1 : 5 |

Die Chromatogramme wurden mit Ninhydrin beziehungsweise nach dem Chlorieren mit Kaliumjodid/Tolidin entwickelt.

Die spezifische optische Drehung wurde mit einem mit Digitalanzeige versehenen photoelektrischen Polarimeter vom Typ Perkin-Elmer 141® gemessen. Das Abdampfen beziehungsweise Entfernen sämtlicher Lösungsmittel erfolgte mit einem Eindampfer vom Typ Rotavapor® von Büchi auf Wasserbädern mit einer Temperatur von höchstens 40 °C.

## Beispiel 1

[Nα-(Benzyloxycarbonyl)-N$^G$-(nitro)]-L-arginyl-[Nε-(benzyloxycarbonyl)]-L-lysyl-L-asparagyl-[β-(benzylester)]-L-valin-p-nitrobenzylester {Z-Arg(NO$_2$)-Lys(Z)-Asp(OBzl)-Val-ONB}

Es wurde zu einer Lösung von 1,73 g (6 Millimol) L-Valin-p-nitrobenzylesterhydrochlorid in 15 cm$^3$ Dimethylformamid 0,84 cm$^3$ (6 Millimol) Triäthylamin und 2,45 g (5 Millimol) [N-(tert.-Butoxycarbonyl)]-L-asparaginsäure-[β-(benzylester)]-[α-(pentafluorphenylester)] zugegeben. Das Reaktionsgemisch wurde 30 Minuten lang bei Zimmertemperatur gerührt beziehungsweise geschüttelt und dann eingedampft und der Rückstand wurde in 30 cm$^3$ Äthylacetat gelöst. Die Lösung wurde mit 15 cm$^3$ einer n Salzsäure, dann mit 15 cm$^3$ einer 5 %-igen Natriumbicarbonatlösung und schließlich mit 15 cm$^3$ Wasser ausgeschüttelt, über wasserfreiem Natriumsulfat getrocknet und dann unter Vakuum eingedampft. Das als Rückstand erhaltene geschützte Dipeptid [N-(tert.-Butoxycarbonyl)-L-asparagyl-[β-(benzylester)]-L-valin-p-nitrobenzylester (R$_f^6$-Wert = 0,9) wurde in 10 cm$^3$ einer 8 n Lösung von salzsaurem Chlorwasserstoff in Dioxan 15 Minuten lang stehengelassen, dann wurde die Lösung mit 40 cm$^3$ wasserfreiem Äther verdünnt und zur Trockne eingedampft. Das als Rückstand erhaltene freie (von der tert.-Butoxycarbonylschutzgruppe befreite) Dipeptid L-Asparagyl-[β-(benzylester)]-L-valin-p-nitrobenzylester (R$_f^2$-Wert = 0,5) wurde in 10 cm$^3$ Dimethylformamid gelöst und die Lösung wurde mit Triäthylamin auf einen pH-Wert von 8 eingestellt und dann mit 3,1 g (5,5 Millimol) [Nα-(tert.-Butoxycarbonyl)-Nε-(benzyloxycarbony)]-L-lysinpentafluorphenylester versetzt. Das Reaktionsgemisch wurde 30 Minuten lang bei Zimmertemperatur gerührt beziehungsweise geschüttelt, wobei der pH-Wert mit Triäthylamin auf 8 gehalten wurde. Dann wurde die Lösung mit 60 cm$^3$ Chloroform verdünnt und zunächst mit 15 cm$^3$ einer n Salzsäure und dann mit 15 cm$^3$ Wasser ausgeschüttelt. Die organische Phase wurde nach dem Trocknen zur Trockne eingedampft und der Rückstand wurde mit wasserfreiem Äther verfestigt. Das erhaltene geschützte Tripeptid [Nα-(tert.-Butoxycarbonyl)-Nε-(benzyloxycarbonyl)]-L-lysyl-L-asparagyl-[β-(benzylester)]-L-valin-p-nitrobenzylester (R$_f^1$-Wert = 0,4) wurde mit 100 cm$^3$ wasserfreiem Äther gefällt, abfiltriert und 2-mal mit Äther gewaschen. Das Produkt wurde in 20 cm$^3$ Dimethylformamid gelöst und die Lösung wurde mit Triäthylamin auf einen pH-Wert von 8 eingestellt und dann mit 3,3 g (7 Millimol) [Nα-(Benzyloxycarbonyl)-N$^G$-(nitro)]-L-arginin-pentafluorphenylester versetzt. Das Reaktionsgemisch wurde 30 Minuten lang bei Zimmertemperatur gerührt beziehungsweise geschüttelt, wobei der pH-Wert der Lösung mit Triäthylamin auf 8 gehalten wurde. Dann wurde das Lösungsmittel abgedampft, der Rückstand mit 50 cm$^3$ Äthanol verrieben, filtriert und 2-mal mit 10 cm$^3$ Äthanol gewaschen. So wurden 3,85 g (Gesamtausbeute von 73 % der Theorie, bezogen auf den Ausgangsstoff [N-(tert.-Butoxycarbonyl)]-L-asparaginsäure-[β-(benzylester)]-[α-(pentafluorphenylester-]) geschütztes Tetrapeptid [Nα-(Benzyloxycarbonyl)-N$^G$-(nitro)]-L-arginyl-[Nε-(benzyloxycarbonyl)]-L-lysyl-L-asparagyl-[β-(benzylester)]-L-valin-p-nitrobenzylester mit einem Schmelzpunkt von 135 bis 148 °C und einem R$_f^2$-Wert von 0,80 erhalten.

## Beispiel 2

[Nα-(Benzyloxycarbonyl)-N$^G$-(nitro)]-L-arginyl-[Nε-(benzyloxycarbonyl)]-L-lysyl-L-asparaginsäure-α,β-dibenzylester {Z-Arg(NO$_2$)-Lys(Z)-Asp(OBzl)-OBzl}

Es wurde ein Gemisch von 1,62 g (3,3 Millimol) [Nα-(tert.-Butoxycarbonyl)-Nε-(benzyloxycarbonyl)]-L-lysinpentafluorphenylester, 1,40 g (4,0 Millimol) L-Asparaginsäure-α,β-dibenzylesterhydrochlorid und 0,98 cm$^3$ (7,0 Millimol) Triäthylamin in 10 cm$^3$ Äthylacetat 1 Stunde lang bei Zimmertemperatur stehengelassen und dann mit 20 cm$^3$ Äthylacetat verdünnt. Das Gemisch wurde zunächst mit 10 cm$^3$ einer n Salzsäure und dann mit 10 cm$^3$ einer 5 %-igen Natriumbicarbonatlösung ausgeschüttelt, über wasserfreiem Natriumsulfat getrocknet und dann unter Vakuum eingedampft. Der Rückstand wurde mit n-Hexan verfestigt, abfiltriert und mit n-Hexan gewaschen. Das erhaltene geschützte Dipeptid [Nα-(tert.-Butoxycarbonyl)-Nε-(benzyloxycarbonyl)]-L-lysyl-L-asparaginsäure-α,β-dibenzylester (Ausbeute : 81,2 % der Theorie, Schmelzpunkt : 92 bis 95 °C und R$_f^2$-Wert = 0,75) wurde 30 Minuten lang mit 30 cm$^3$ einer 4 n Lösung von Chlorwasserstoff in Dioxan behandelt und dann zur Trockne eingedampft. Das freie (von

19

der tert.-Butoxycarbonylschutzgruppe befreite) Dipeptid [Nε-(Benzyloxycarbonyl)]-L-lysyl-L-asparaginsäure-α,β-dibenzylester (R$_f$²-Wert = 0,10) wurde in 10 cm³ Dimethylformamid gelöst, die Lösung wurde mit 0,35 cm³ Triäthylamin neutralisiert und die erhaltene Suspension wurde zum durch Lösen von 10,6 g (3,0 Millimol) [Nα-(Benzyloxycarbonyl)-N$^G$-(nitro)]-L-arginin und 0,42 cm³ (3,0 Millimol) Triäthylamin in 5 cm³ Dimethylformamid, Kühlen dieser Lösung auf −10 °C und deren tropfenweises Versetzen bei dieser Temperatur mit 0,36 cm³ (3,0 Millimol) Pivaloylchlorid hergestellten gemischten Anhydrid in Lösung 5 Minuten nach seiner Herstellung bei −10 °C zugegeben. Das Reaktionsgemisch wurde bei O °C noch 30 Minuten gerührt beziehungsweise geschüttelt, dann über Nacht stehengelassen und am nächsten Tag zur Trockne eingedampft. Der Rückstand wurde in 50 cm³ Chloroform gelöst und die Lösung wurde mit 10 cm³ einer n Salzsäure, dann mit 10 cm³ einer 5 %-igen Natriumbicarbonatlösung und schließlich mit 10 cm³ Wasser ausgeschüttelt. Die organische Phase wurde nach dem Trocknen zur Trockne eingedampft und der erhaltene Rückstand wurde mit einem Gemisch aus Äther und n-Hexan im Volumverhältnis von 1 : 1 verfestigt. So wurden 1,82 g (84 % der Theorie) geschütztes Tripeptid [Nα-(Benzyloxycarbonyl)-N$^G$-(nitro)]-L-arginyl-[Nε-(benzyloxycarbonyl)]-L-lysyl-L-asparaginsäure-α,β-dibenzylester mit einem R$_f$²-Wert von 0,70 erhalten.

## Beispiel 3

[Nα-(Benzyloxycarbonyl)-N$^G$-(nitro)]-L-arginyl-[Nε-(benzyloxycarbonyl)]-L-lysyl-L-aminosuccinyl-L-valin {Z-Arg(NO$_2$)-Lys(Z)-Asu-Val-OH}

Es wurden 2,51 g (12 Millimol) L-Valin-tert.-butylester-hydrochlorid in 50 cm³ Chloroform gelöst. Die Lösung wurde mit 3,86 g (10 Millimol) [N-(tert.-Butoxycarbonyl)]-L-asparaginsäure-[β-(tert.-butylester)]-[α-(N$_{succ}$-oxy-succinimidester)] {Boc-Asp(O$^t$Bu)-OSu} und 1,68 cm³ (12 Millimol) Triäthylamin versetzt. Am nächsten Tag wurde die Lösung zunächst 3-mal mit je 10 cm³ einer n Salzsäure und dann 3-mal mit je 10 cm³ einer 5 %-igen Natriumbicarbonatlösung ausgeschüttelt. Nach dem Trocknen wurde das Lösungsmittel abgedampft. Das als Rückstand erhaltene geschützte Dipeptid [N-(tert.-Butoxycarbonyl)]-L-asparagyl-[β-(tert.-butylester)]-L-valin-tert.-butylester (R$_f$²-Wert = 0,80) wurde in 30 cm³ einer 5 n Lösung von Bromwasserstoff in Essigsäure gelöst und die Lösung wurde 1 Woche lang stehengelassen. Dann wurde das Reaktionsgemisch zur Trockne eingedampft und der Rückstand mit wasserfreiem Äther verfestigt. So wurden 2,65 g (98,2 % der Theorie) L-Aminosuccinyl-L-valinhydrobromid (R$_f$⁴-Wert = 0,15) erhalten und diese auf die im Beispiel 1 angebebene Weise weiter acyliert. Die Daten des geschützten Tetrapeptides [Nε-(Benzyloxycarbonyl)-N$^G$-(nitro)]-L-arginyl-[Nε-(benzyloxycarbonyl)]-L-lysyl-L-aminosuccinyl-L-valin wie die der anderen geschützten Peptide sind in der unten folgenden Tabelle 5 enthalten.

## Beispiel 4

L-Arginyl-L-lysyl-L-asparagyl-L-valin

Es wurden 2,25 g (2,22 Millimol) wie im Beispiel 1 beschrieben erhaltenes [Nα-(Benzyloxycarbonyl)-N$^G$-(nitro)]-L-arginyl-[Nε-(benzyloxycarbonyl)]-L-lysyl-L-asparagyl-[β-(benzylester)]-L-valin-p-nitrobenzylester in 50 cm³ einer 90 %-igen Essigsäure suspendiert. Die Suspension wurde mit 1 g von 5 Gew.-% Palladium auf Aktivkohle versetzt und durch das Gemisch wurde 14 Stunden lang Wasserstoff geleitet. Dann wurde der Katalysator abfiltriert und 2-mal mit je 10 cm³ einer 90 %-igen Essigsäure gewaschen und das Filtrat wurde mit der Waschflüssigkeit vereinigt und dann zur Trockne eingedampft. Der Rückstand wurde mit Wasser und Äthanol erneut eingedampft, dann in 2 cm³ Wasser gelöst und mit 30 cm³ Äthanol versetzt. Die erhaltene Suspension wurde filtriert und der Niederschlag wurde mit Äthanol gewaschen. So wurde 0,92 g (80 % der Theorie) Monoacetat des freien Tetrapeptides L-Arginyl-L-lysyl-L-asparagyl-L-valin mit einem [α]$_D$²²-Wert von −24,8° (c = 1,0, in einer 10 %-igen Essigsäure) und einem R$_f$⁸-Wert von 0,10 erhalten.

Aminosäureanalyse :

| | | |
|---|---|---|
| L-Lysin | = 1,05 [1,00] | |
| L-Arginin | = 0,95 [1,00] | |
| L-Asparaginsäure | = 1,04 [1,00] | und |
| L-Valin | = 0,95 [1,00] | |

Nach den Verfahrensweisen der Beispiele 1, 2, 3 beziehungsweise 4 wurden durch Umsetzen von äquimolaren Mengen der entsprechend gewählten Ausgangsstoffe weitere erfindungsgemäße Verbindungen, die zusammen mit denen der Beispiele 1 bis 4 in den folgenden Tabellen 5 und 6 zusammengestellt sind, hergestellt. Dabei betrifft die Tabelle 5 erfidungsgemäße geschützte Peptide und die Tabelle 6 erfindungsgemäße freie Peptide beziehungsweise Amide und Ester von solchen.

(Siehe Tabellen Seite 21 ff.)

Tabelle 5

Herstellung und Eigenschaften von geschützten Peptiden

| Peptid | | Ausbeute in % | | Verfahrensweise des Beispieles Nr. | Schmelzpunkt in °C | $R_f$-Wert (Lösungsmittelgemisch) | Reinigung |
|---|---|---|---|---|---|---|---|
| Beispiel-Nr. | Bezeichnung | Gesamtausbeute | Durchschnittsausbeute je Schritt | | | | |
| 1 | $[N^\alpha$-(Benzyloxycarbonyl)-$N^G$-(nitro)]-L-arginyl-[$N^\epsilon$-(benzyloxycarbonyl)]-L-lysyl-L-asparagyl-[ß-(benzylester)]-L-valin-p-nitrobenzylester | 73 | 90 | 1 | 135 bis 148 | 0,8 (2) | 80%-ige Essigsäure |
| 2 | $[N^\alpha$-(Benzyloxycarbonyl)-$N^G$-(nitro)]-L-arginyl-[$N^\epsilon$-(benzyloxycarbonyl)]-L-lysyl-L-asparaginsäure-$\alpha$,ß-dibenzylester | 68 | 82 | 2 | amorph | 0,70 (2) | Äther/n-Hexan im Volumverhältnis von 1:2 (Waschen) |
| 3 | $[N^\alpha$-(Benzyloxycarbonyl)-$N^G$-(nitro)]-L-arginyl-[$N^\epsilon$-(benzyloxycarbonyl)]-L-lysyl-L-aminosuccinyl-L-valin | 62 | 85 | 3 und 1 | 94 (unter Zersetzung) | 0,30 (3) | Äthanol/Äther im Volumverhältnis von 1 : 4 |
| 5 | $[N^\alpha,N^G,N^G$-Tri-(benzyloxycarbonyl)]-L-arginyl-[$N^\epsilon$-(benzyloxycarbonyl)]-L-lysyl-L-asparagyl-[ß-(benzylester)]-L-valinamid {Z-Arg(Z$_2$)-Lys(Z)-Asp(OBzl)-Val-NH$_2$} | 52 | 81 | 1 | 214 bis 216 | 0,70 (2) | Äthanol (Auskochen) |
| 6 | $[N^\alpha,N^\epsilon$-Di-(benzyloxycarbonyl)]-L-lysyl-[$N^G$-(nitro)]-L-arginyl-L-asparagyl-[ß-(benzylester)]-L-valin-p-nitrobenzylester {Z-Lys(Z)-Arg(NO$_2$)-Asp(OBzl)-Val-ONB} | 78 | 92 | 1 | 117 bis 120 | 0,60 (2) | Äthanol/Äther im Volumverhältnis von 1 : 2 |

Fortsetzung der Tabelle 5

| Beispiel-Nr. | Peptid Bezeichnung | Ausbeute in % Gesamtausbeute | Ausbeute in % Durchschnittsausbeute je Schritt | Verfahrensweise des Beispieles Nr. | Schmelzpunkt in °C | $R_f$-Wert | (Lösungsmittelgemisch) | Reinigung |
|---|---|---|---|---|---|---|---|---|
| 7 | $[N^\alpha$-(Benzyloxycarbonyl)-$N^G$-(nitro)]-L-arginyl-$[N^\epsilon$-(benzyloxycarbonyl)]-L-lysyl-L-asparagyl-[ß-(benzylester)]-L-valinmethylester $\{Z$-Arg(NO$_2$)-Lys(Z)-Asp(OBzl)-Val-OMe$\}$ | 52 | 80 | 2 | 125 bis 135 | 0,60 (2) | | Äthanol |
| 8 | $[N^\alpha$-(Benzyloxycarbonyl)]-L-pyroglutamyl-$[N^G$-(nitro)]-L-arginyl-$[N^\epsilon$-(benzyloxycarbonyl)]--L-lysyl-L-asparagyl-[ß-(benzylester)]-L--valin-p-nitrobenzylester $\{Z$-Glu-Arg(NO$_2$)-Lys(Z)-Asp(OBzl)-Val-ONB$\}$ | 59 | 84 | 1 | 166 bis 172 | 0,80 (3) | | Äthanol |
| 9 | $[N^\alpha$-(Benzyloxycarbonyl)]-L-pyroglutamyl--$[N^G$-(nitro)]-L-arginyl-$[N^\epsilon$-(benzyloxycarbonyl)]-L-lysyl-L-asparaginsäure-$\alpha$,ß-dibenzylester $\{Z$-Glu-Arg(NO$_2$)-Lys(Z)-Asp(OBzl)-OBzl$\}$ | 60 | 85 | 1 | 100 bis 104 | 0,60 (2) | | Äthanol/Äther im Volumverhältnis von 1 : 2 |

0 067 425

Fortsetzung der Tabelle 5

| Peptid | | Ausbeute in % | | Verfahrensweise des Beispieles Nr. | Schmelzpunkt in °C | $R_f$-Wert | (Lösungsmittel gemisch) | Reinigung |
|---|---|---|---|---|---|---|---|---|
| Beispiel-Nr. | Bezeichnung | Gesamtausbeute | Durchschnittsausbeute je Schritt | | | | | |
| 10 | $[N^\alpha, N^G, N^G$-Tri-(benzyloxycarbonyl)]-L-arginyl- $[N^\epsilon$-(benzyloxycarbonyl)]-L-lysyl-L-asparaginyl-L-valinbenzylester {Z-Arg($Z_2$)-Lys(Z)-Asn-Val-OBzl} | 41 | 74 | 1 | 193 bis 195 | 0,7 (3) | | Äthanol (Auskochen) |
| 11 | [N-(benzyloxycarbonyl)]-L-alanyl-$[N^\epsilon$-(benzyloxycarbonyl)]-L-lysyl-L-asparagyl-[ß-(benzylester)]-L-valin-p-nitrobenzylester {Z-Ala-Lys-(Z)-Asp(OBzl)-Val-ONB} | 80 | 93 | 1 | 174 bis 176 | 0,80 (1) | | Äthanol |
| 12 | $[N^\alpha$-(Benzyloxycarbonyl)-$N^G$-(nitro)]-L-arginyl-L-alanyl-L-asparagyl-[ß-(benzylester)]- -L-valin-p-nitrobenzylester {Z-Arg($NO_2$)-Ala-Asp(OBzl)-Val-ONB} | 60 | 85 | 1 | 184 bis 186 | 0,30 (1) | | 90%-iges Äthanol |

Fortsetzung der Tabelle 5

| Peptid | | Ausbeute in % | | Ver-fahrens-weise des Bei-spieles Nr. | Schmelz-punkt in °C | $R_f$-Wert | (Lö-sungs-mit-tel ge-misch) | Rei-ni-gung |
|---|---|---|---|---|---|---|---|---|
| Bei-spiel-Nr. | Bezeichnung | Gesamt-aus-beu-te | Durch-schnitts-ausbeute je Schritt | | | | | |
| 13 | $[N^\alpha$-(Benzyloxycarbonyl)-$N^G$-(nitro)]-L-argi-nyl-$[N^\epsilon$-(benzyloxycarbonyl)]-L-lysyl-L-ala-nyl-L-valin-p-nitrobenzylester $\{Z\text{-}Arg(NO_2)\text{-}Lys(Z)\text{-}Ala\text{-}Val\text{-}ONB\}$ | 68 | 87 | 1 | 157 bis 159 | 0,45 | (1) | 60%-ige Essigsäure |
| 14 | $[N^\alpha$-(Benzyloxycarbonyl)-$N^G$-(nitro)]-L-argi-nyl-$[N^\epsilon$-(benzyloxycarbonyl)]-L-lysyl-L-aspa-ragyl-$[\beta$-(benzylester)]-L-alanin-p-nitroben-zylester $\{Z\text{-}Arg(NO_2)\text{-}Lys(Z)\text{-}Asp(OBzl)\text{-}Ala\text{-}ONB\}$ | 50 | 80 | 1 | 124 bis 126 | 0,45 | (1) | 60%-ige Essigsäure |
| 15 | $[N^\alpha$-(Benzyloxycarbonyl)-$N^G$-(nitro)]-L-argi-nyl-$[N^\epsilon$-(benzyloxycarbonyl)]-L-lysyl-L-as-paragyl-$[\beta$-(benzylester)]-L-isoleucinbenzyl-ester $\{Z\text{-}Arg(NO_2)\text{-}Lys(Z)\text{-}Asp(OBzl)\text{-}Ile\text{-}OBzl\}$ | 64 | 86 | 2 | 136 bis 138 | 0,70 | (2) | Äthanol |

0 067 425

Fortsetzung der Tabelle 5

| Peptid | | Ausbeute in % | | Ver-fah-rens-weise des Bei-spieles Nr. | Schmelz-punkt in °C | $R_f$-Wert | (Lö-sungs-mit-tel ge-misch) | Rei-ni-gung |
|---|---|---|---|---|---|---|---|---|
| Bei-spiel-Nr. | Bezeichnung | Gesamt-aus-beu-te | Durch-schnitts-ausbeute je Schritt | | | | | |
| 16 | [N$^\alpha$-(Benzyloxycarbonyl)-N$^G$-(nitro)]-L-argi-nyl-L-asparagyl-[ß-(benzylester)]-[N$^\epsilon$-(benzyloxy-carbonyl)]-L-lysyl-L-valinbenzylester {Z-Arg(NO$_2$)-Asp(OBzl)-Lys(Z)-Val-OBzl} | 42 | 75 | 2 | 136 bis 141 | 0,65 | (2) | Äthylacetat |
| 17 | [N$^\alpha$-(Benzyloxycarbonyl)]-L-pyroglutamyl-[N$^G$-(nitro)]-L-arginyl-[N$^\epsilon$-(benzyloxycar-bonyl)]-L-lysyl-L-asparagyl-[ß-(benzylester)]-L-valyl-L-tyrosin-benzyläther-p-nitrobenzyl-ester {Z-Glu-Arg(NO$_2$)-Lys(Z)-Asp(OBzl)-Val-Tyr(Bzl)-ONB} | 68 | 87 | 1 | 199 bis 205 | 0,80 | (3) | 60%-ige Essigsäure |
| 18 | [N$^\alpha$-(Benzyloxycarbonyl)-N$^G$-(nitro)]-L-argi-nyl-[N$^\epsilon$-(benzyloxycarbonyl)]-L-lysyl-L-gluta-myl-[δ-(p-chlorbenzylester]-L-valinbenzyl-ester {Z-Arg(NO$_2$)-Lys(Z)-Glu(OBzl-4-Cl-Val-OBzl} | 39 | 73 | 2 | 124 bis 127 | 0,65 | (2) | Äthylacetat |

0 067 425

Tabelle 6

Eigenschaften der freien Peptide

| Peptid | | | Ausbeute in % | $R_f$-Wert Lösngs-mittelge-misch | $[\alpha]_D^{22}$-Wert [*)] |
|---|---|---|---|---|---|
| Bei-spiel Nr. | Bezeichnung | als | | | |
| 4 | L-Arginyl-L-lysyl-L-asparagyl-L--valin | Monoace-tat [**)] | 80 | 0,10 (8) | -24,8 |
| 19 | L-Arginyl-L-lysyl-L-asparaginsäure | Monoacetat | 83 | 0,20 (7) | 0 |
| 20 | L-Arginyl-L-lysyl-L-asparagyl-L-va-linamid | Diacetat | 84,3 | 0,20 (7) | -26,5 |
| 21 | L-Lysyl-L-arginyl-L-asparagyl-L-valin | Monoace-tat [**)] | 77 | 0,30 (5) | -39,5 |
| 22 | L-Arginyl-L-lysyl-L-asparagyl-L-valin-methylester | Di-acetat | 84,6 | 0,35 (5) | -28,3 |
| 23 | L-Pyroglutamyl-L-arginyl-L-lysyl-L--asparagyl-L-valin | Monoace-tat [**)] | 93 | 0,40 (5) | -51,3 |
| 24 | L-Pyroglutamyl-L-arginyl-L-lysyl-L--asparagin | Monoace-tat [**)] | 70 | 0,35 (5) | -31,4 |

Fortsetzung der Tabelle 6

| Peptid | | | Ausbeute in % | $R_f$-Wert | Lösngs-mittelge-misch | $[\alpha]_D^{22}$-Wert [*] |
|---|---|---|---|---|---|---|
| Bei-spiel Nr. | Bezeichnung | als | | | | |
| 25 | L-Arginyl-L-lysyl-L-asparaginyl-L-valin | Diacetat | 79,5 | 0,10 | (5) | − 23,0 |
| 26 | L-Alanyl-L-lysyl-L-asparagyl-L-valin | Monoacetat-hydrat | 90 | 0,35 | (5) | −38,6 |
| 27 | L-Arginyl-L-alanyl-L-asparagyl-L-valin | freie Base | 87 | 0,45 | (5) | −37,9 |
| 28 | L-Arginyl-L-lysyl-L-alanyl-L-valin | Triacetat | 88,5 | 0,45 | (5) | −34,6 |
| 29 | L-Arginyl-L-lysyl-L-aminosuccinyl--L-valin | Diacetat | 68 | 0,15 | (5) | −31,0 |
| 30 | L-Arginyl-L-lysyl-L-asparagyl-L-alanin | Monoacetat | 88 | 0,25 | (5) | −19,9 |
| 31 | L-Arginyl-L-lysyl-L-asparagyl-L-iso-leucin | Monoacetat | 48,6 | 0,15 | (5) | −19,5 |
| 32 | L-Arginyl-L-asparagyl-L-lysyl-L-valin | Monoacetat | 89,2 | 0,25 | (7) | −19,4 |

Fortsetzung der Tabelle 6

| Peptid | | | Ausbeute in %| $R_f$-Wert Lösungsmittelgemisch | | $[\alpha]_D^{22}$-Wert *) |
|---|---|---|---|---|---|---|
| Beispiel Nr. | Bezeichnung | als | | | | |
| 33 | L-Pyroglutamyl-L-arginyl-L-lysyl-L--asparagyl-L-valyl-L-tyrosin | freie Base **) | 80,0 | 0,20 | (8) | -51,5 |
| 34 | L-Arginyl-L-lysyl-L-glutamyl-L-valin | Monoacetat | 97 | 0,30 | (7) | -21,4 |

*) c = 1, in 10 %- iger Essigsäure
**) chromatographiert an einer mit Kieselgel SI-100® gefüllten Säule mit Wasser und Methanol im Volumverhältnis von 1 : 1

0 067 425

**Patentansprüche**

1. Peptide der Formeln

| | |
|---|---|
| Arg-Lys-Asp | I, |
| Arg-Lys-Asp-Val | II, |
| Arg-Lys-Asn-Val | III, |
| Arg-Lys-Asu-Val | IV, |
| Arg-Lys-Ala-Val | V, |
| Arg-Lys-Asp-Ala | VI, |
| Arg-Lys-Asp-Ile | VII, |
| Arg-Lys-Glu-Val | VIII, |
| Arg-Ala-Asp-Val | IX, |
| Arg-Asp-Lys-Val | X, |
| Ala-Lys-Asp-Val | ·XI, |
| Lys-Arg-Asp-Val | XII, |
| < Glu-Arg-Lys-Asp | XIII, |
| < Glu-Arg-Lys-Asp-Val | XIV, |
| < Glu-Arg-Lys-Asp-Val-Tyr | XV, |

sowie deren Salze, Komplexe, Amide und Alkylester mit 1 bis 5 Kohlenstoffatom(en) im Alkylteil derselben, gegebenenfalls substituiert mit in der Peptidchemie üblicherweise verwendeten Schutzgruppen.

2. Peptide nach Anspruch 1, dadurch gekennzeichnet, daß ihre Alkylester solche mit 1 bis 4, insbesondere 1 oder 2, Kohlenstoffatom(en) im Alkylteil sind.

3. Verfahren zur Herstellung der Verbindungen nach Anspruch 1 oder 2, dadurch gekennzeichnet, daß man in in der Peptidchemie an sich bekannter Weise aus den entsprechenden geschützten Aminosäuren beziehungsweise ihren Salzen, Komplexen, Amiden beziehungsweise Alkylestern die geschützten Peptide beziehungsweise ihre Salze, Komplexe, Amide beziehungsweise Alkyl-ester synthetisiert und in an sich bekannter Weise gegebenenfalls von diesen die Schutzgruppe(n) abspaltet und/oder gegebenenfalls die, gegebenenfalls geschützten, Peptide der allgemeinen Formeln, I, II, III, IV, V, VI, VII, VIII, IX, X, XI, XII, XIII, XIV beziehungsweise XV in Salze oder Komplexe überführt beziehungsweise aus den Salzen die freien Basen freisetzt und/oder sie in andere Salze überführt.

4. Verfahren nach Anspruch 3, dadurch gekennzeichnet, daß man die C-endständige Aminosäure beziehungsweise ein, gegebenenfalls an der Seitenfunktion geschütztes, die C-endständige Aminosäure aufweisendes Peptidfragment des herzustellenden Peptides beziehungsweise Salzes, Komplexes, Amides beziehungsweise Alkylesters desselben beziehungsweise ein Amid beziehungsweise einen Alkylester mit 1 bis 5 Kohlenstoffatom(en) beziehungsweise ein an der Carboxylgruppe in sonstiger Weise geschütztes Derivat dieser C-endständigen Aminosäure beziehungsweise dieses Peptidfragmentes mit einem an ihrem beziehungsweise seinem endständigen Stickstoffatom und gegebenenfalls an der Seitenfunktion geschützten und an der Carboxylgruppe aktivierten Derivat der in der Aminosäuresequenz des herzustellenden Peptides beziehungsweise Salzes, Komplexes, Amides beziehungsweise Alkylesters desselben vor ihr stehenden Aminosäure oder einem an seinem endständigen Stickstoffatom und gegebenenfalls an der Seitenfunktion geschützten und an der Carboxylgruppe aktivierten in der Aminosäuresequenz des herzustellenden Peptides beziehungsweise Salzes, Komplexes, Amides beziehungsweise Alkylesters desselben vor ihr stehende Aminosäuren aufweisenden Peptidfragmentderivat acyliert und gegebenenfalls in [einer] nächsten Stufe(n) mit dem erhaltenen am endständigen Stickstoffatom geschützten Peptidzwischenprodukt und dem beziehungsweise den durch [eine] etwaige weitere derartige Acylierung(en) erhaltenen am endständigen Stickstoffatom geschützten weiteren Peptidzwischenprodukt(en) nach in an sich bekannter Weise erfolgendem Entfernen der Schutzgruppe des zu acylierenden endständigen Stickstoffatomes eine weitere beziehungsweise weitere Acylierung(en) mit beziehungsweise jeweils mit einem an ihrem beziehungsweise seinem endständigen Stickstoffatom geschützten und an der Carboxylgruppe aktivierten Derivat der in der Aminosäuresequenz des herzustellenden Peptides beziehungsweise Salzes, Komplexes, Amides beziehungsweise Alkylesters desselben vor ihr stehenden Aminosäure oder einem an seinem endständigen Stickstoffatom und gegebenenfalls an der Seitenfunktion geschützten und an der Carboxylgruppe aktivierten in der Aminosäuresequenz des herzustellenden Peptides beziehungsweise Salzes, Komplexes, Amides beziehungsweise Alkylesters desselben vor ihr stehende Aminosäuren aufweisenden Peptidfragmentderivat vornimmt, wobei man so viele Acylierungen durchführt, wie es zum Erreichen der gewünschten Aminosäuresequenz erforderlich ist, und gegebenenfalls danach in an sich bekannter Weise vom erhaltenen geschützten Peptid die Schutzgruppe des endständigen Stickstoffatomes und die etwaige(n) andere(n) Schutzgruppe(n) entfernt und gegebenenfalls in an sich bekannter Weise das erhaltene geschützte oder von der beziehungsweise den Schutzgruppe(n) befreite Peptid der allgemeinen Formeln I, II, III, IV, V, VI, VII, VIII, IX, X, XI, XII, XIII, XIV beziehungsweise XV in ein Säureadditionssalz oder einen Komplex überführt beziehungsweise gegebenenfalls das erhaltene Säureadditionssalz des Peptides der

allgemeinen Formeln I, II, III, IV, V, VI, VII, VIII, IX, X, XI, XII, XIII, XIV beziehungsweise XV in das Peptid der allgemeinen Formeln I, II, III, IV, V, VI, VII, VIII, IX, X, XI, XII, XIII, XIV beziehungsweise XV oder in ein anderes Säureadditionssalz überführt.

5. Verfahren nach Anspruch 3 oder 4, dadurch gekennzeichnet, daß man zum Acylieren als aktiviertes Derivat einen aktiven Ester oder ein gemischtes Anhydrid der acylierenden Aminosäure beziehungsweise des acylierenden Peptidfragmentes verwendet.

6. Arzneimittel, gekennzeichnet durch einen Gehalt an 1 oder mehr Verbindung(en) nach Anspruch 1 oder 2 als Wirkstoff(en), zweckmäßigerweise zusammen mit 1 oder mehr üblichen pharmazeutischen Konfektionierungsmitteln(n).

**Claims**

1. Peptides having the formulae

| | |
|---|---|
| Arg-Lys-Asp | I, |
| Arg-Lys-Asp-Val | II, |
| Arg-Lys-Asn-Val | III, |
| Arg-Lys-Asu-Val | IV, |
| Arg-Lys-Ala-Val | V, |
| Arg-Lys-Asp-Ala | VI, |
| Arg-Lys-Asp-Ile | VII, |
| Arg-Lys-Glu-Val | VIII, |
| Arg-Ala-Asp-Val | IX, |
| Arg-Asp-Lys-Val | X, |
| Ala-Lys-Asp-Val | XI, |
| Lys-Arg-Asp-Val | XII, |
| < Glu-Arg-Lys-Asp | XIII, |
| < Glu-Arg-Lys-Asp-Val | XIV, |
| < Glu-Arg-Lys-Asp-Val-Tyr | XV, |

as well as their salts, complexes, amides and alkyl esters having from 1 to 5 carbon atom(s) in the alkyl part of them, optionally substituted by protective groups usually used in the peptide chemistry.

2. Peptides according to claim 1, characterized in that their alkyl esters are such having from 1 to 4, particularly 1 or 2, carbon atom(s) in the alkyl part.

3. A process for preparing the compounds according to claim 1 or 2, characterized in that one synthetizes in a manner known per se in the peptide chemistry from the corresponding protected amino acids or their salts, complexes amides or alkyl esters, respectively, the protected peptides or their salts, complexes, amides or alkyl esters, respectively, and in a manner known per se optionally one splits off from these the protective group(s) and/or optionally one converts the peptides having the general formulae I, II, III, IV, V, VI, VII, VIII, IX, X, XI, XII, XIII, XIV or XV, respectively, optionally protected into salts or complexes or one liberates from the salts the free basis and/or one converts them into other salts, respectively.

4. A process according to claim 3, characterized in that one acylates the C-terminal amino acid or a peptide fragment of the peptide to be prepared or of the salt, complex, amide or alkyl ester of it, respectively, containing the C-terminal amino acid, optionally protected in the side function or an amide or an alkyl ester having from 1 to 5 carbon atom(s), respectively, or a derivative of this C-terminal amino acid or of this peptide fragment protected in another way on the carboxylic group with a derivative protected on its terminal nitrogen atom and optionally in the side function and activated on the carboxyl group of the amino acid preceeding in the amino acid sequence of the peptide or salt, complex, amide or alkyl ester, respectively, of it to be prepared or with a peptide fragment derivative containing the amino acids preceeding in the amino acid sequence of the peptide to be prepared or of its salt, complex, amide or alkyl ester, respectively, and protected on its terminal nitrogen atom and optionally in the side function and activated on the carboxyl group and optionally in [one] next step(s) one carries out a further or further acylation(s) with the obtained peptide intermediate protected on the terminal nitrogen atom and the further peptide intermediate(s) protected on the terminal nitrogen atom obtained by [a] possible further such acylation(s) after elimination in a manner known per se of the protective group of the terminal nitrogen atom to be acylated with the or each of the derivative(s) protected on its terminal nitrogen atom and activated on its carboxyl group of the amino acid preceeding in the amino acid sequence of the peptide or salt, complex, amide or alkyl ester, respectively, of it, to be prepared or with a peptide fragment protected on its terminal nitrogen atom and optionally in the side function and activated on the carboxyl group and containing the amino acids preceeding in the amino acid sequence of the peptide or salt, complex, amide or alkyl ester, respectively, of it to be prepared carrying out so much acylations as it is necessary to attain the desired amino acid sequence and optionally one eliminates in a manner known per se from the obtained protected peptide the protective group of the terminal nitrogen atom and the possible other protective group(s) and optionally in a manner known per se one converts the obtained peptide protected or liberated from the protective group(s) and having the general formulae I, II, III, IV, V, VI, VII, VIII, IX, X, XI, XII, XIII, XIV or XV, respectively, into an acid addition salt or into a complex or optionally one converts the obtained acid addition salt of the peptide having the general formulae I, II, III, IV, V, VI, VII, VIII, IX, X, XI, XII, XIII, XIV or XV, respectively, into the peptide having the general formulae I, II, III, IV, V, VI, VII, VIII, IX, X, XI, XII, XIII, XIV or XV, respectively, or into another acid addition salt.

5. A process according to claim 3 or 4, characterized in that one uses for the acylation as an activated derivative an active ester or a mixed anhydride of the amino acid to be acylated or of the peptide fragment to be acylated.

6. Medicaments, characterized by a content of 1 or more compound(s) according to claim 1 or 2 as active principle(s), usefully together with 1 or more usual pharmaceutical processing agent(s).

**Revendications**

1. Peptides correspondant aux formules ci-après :

| | |
|---|---|
| Arg-Lys-Asp | I, |
| Arg-Lys-Asp-Val | II, |
| Arg-Lys-Asn-Val | III, |
| Arg-Lys-Asu-Val | IV, |
| Arg-Lys-Ala-Val | V, |
| Arg-Lys-Asp-Ala | VI, |
| Arg-Lys-Asp-Ile | VII, |
| Arg-Lys-Glu-Val | VIII, |
| Arg-Ala-Asp-Val | IX, |
| Arg-Asp-Lys-Val | X, |
| Ala-Lys-Asp-Val | XI, |
| Lys-Arg-Asp-Val | XII, |
| < Glu-Arg-Lys-Asp | XIII, |
| < Glu-Arg-Lys-Asp-Val | XIV, |
| < Glu-Arg-Lys-Asp-Val-Tyr | XV, |

ainsi que leurs sels, complexes, amides et esters d'alkyle contenant 1 à 5 atomes de carbone dans leur fragment alkyle, éventuellement substitués par des groupes protecteurs habituellement utilisés dans la chimie des peptides.

2. Peptides selon la revendication 1, caractérisés par le fait que leurs esters d'alkyle sont des esters contenant 1 à 4, en particulier 1 ou 2 atomes de carbone dans le fragment alkyle.

3. Procédé de préparation des composés selon l'une des revendications 1 et 2, caractérisé par le fait qu'en partant, de manière en elle-même connue dans la chimie des peptides, des aminoacides protégés correspondants ou de leurs sels, complexes, amides ou esters d'alkyle, on synthétise les peptides protégés ou leurs sels, complexes, amides ou esters d'alkyle et que de manière en elle-même connue, on élimine éventuellement de ceux-ci le ou les groupes protecteurs et/ou qu'éventuellement, on convertit en sels ou complexes les peptides de formules générales I, II, III, IV, V, VI, VII, VIII, IX, X, XI, XII, XIII, XIV ou XV, ou qu'en partant des sels on libère les bases libres et/ou que l'on convertit ces sels en d'autres sels.

4. Procédé selon la revendication 3, caractérisé par le fait que l'on acyle l'aminoacide de l'extrémité C ou un fragment peptide, éventuellement protégé sur la fonction latérale, présentant l'aminoacide d'extrémité C, du peptide à préparer ou d'un sel, complexe, amide ou ester d'alkyle de celui-ci, ou une amine ou un ester d'alkyle contenant 1 à 5 atomes de carbone ou un dérivé, protégé sur le groupe carboxyle d'une autre façon, de cet aminoacide d'extrémité C ou de ce fragment de peptide, avec un dérivé, protégé sur son atome d'azote terminal et éventuellement sur la fonction latérale et activé sur le groupe carboxyle, de l'aminoacide placé avant lui dans la séquence d'aminoacides du peptide à préparer ou du sel, complexe, amide ou ester d'alkyle de celui-ci, ou avec un dérivé de fragment de peptide, protégé sur son atome d'azote terminal et éventuellement sur la fonction latérale et activé sur le groupe carboxyle, présentant des aminoacides placés avant lui dans la séquence d'aminoacides du peptide à préparer ou du sel, complexe, amide ou ester d'alkyle de celui-ci et qu'éventuellement, en une ou des étapes suivantes, avec l'intermédiaire peptide obtenu, protégé sur l'atome d'azote terminal et le ou les autres intermédiaires peptides protégés sur l'atome d'azote terminal, obtenus par une ou des acylations supplémentaires éventuelles de ce genre, après élimination effectuée de façon connue du groupe protecteur de l'atome d'azote terminal à acyler, on effectue une autre ou d'autres acylations, avec, ou chaque fois avec un dérivé, protégé sur son atome d'azote terminal et activé sur le groupe carboxyle, de l'aminoacide placé avant lui dans la séquence d'aminoacides du peptide à préparer ou du sel, complexe, amide ou ester d'alkyle de celui-ci, ou avec un dérivé de fragment peptide protégé sur son atome d'azote terminal et éventuellement sur la fonction latérale et activé sur le groupe carboxyle, présentant des aminoacides placés avant lui dans la séquence d'aminoacides du peptide à préparer ou du sel, complexe, amide ou ester d'alkyle de celui-ci, en effectuant autant d'acylations qu'il est nécessaire pour arriver à la séquence désirée d'aminoacides, et qu'éventuellement ensuite, de manière en elle-même connue, on élimine du peptide protégé obtenu le groupe protecteur de l'atome d'azote terminal et le ou les autres groupes protecteurs éventuels et qu'éventuellement, de manière en elle-même connue, on convertit le peptide obtenu, protégé ou débarrassé du ou des groupes protecteurs, de formules générales I, II, III, IV, V, VI, VII, VIII, IX, X, XI, XII, XIII, XIV ou XV, en un sel d'addition d'acide ou en un complexe, ou qu'éventuellement, on convertit le sel d'addition d'acide obtenu du peptide de formules générales I, II, III, IV, V, VI, VII, VIII, IX, X, XI, XII, XIII, XIV ou XV, en peptide de formules générales I, II, III, IV, V, VI, VII, VIII, IX, X, XI, XII, XIII, XIV ou XV ou en un autre sel d'addition d'acide.

5. Procédé selon l'une des revendications 3 et 4, caractérisé par le fait que pour l'acylation, on utlise comme dérivé activé un ester actif ou un anhydride mixte de l'aminoacide assurant l'acylation ou du fragment de peptide assurant l'acylation.

6. Médicament caractérisé par une teneur en un ou plusieurs composés selon l'une des revendications 1 et 2, avantageusement en même temps qu'un ou plusieurs agents de conditionnement pharmaceutiques usuels.